# EUROPEAN PATENT SPECIFICATION

(11) **EP 3 523 038 B1**
(45) Date of publication and mention of the grant of the patent: **03.03.2021**
(21) Application number: 17784857.9
(22) Date of filing: 05.10.2017
(51) Int. Cl.: B01L 3/00

(54) **METHOD FOR CONTROLLING AN ANALYSIS DEVICE AND ANALYSIS SYSTEM**
VERFAHREN ZUR STEUERUNG EINER ANALYSEVORRICHTUNG UND ANALYSESYSTEM
PROCÉDÉ DE COMMANDE D'UN APPAREIL D'ANALYSE ET SYSTÈME D'ANALYSE

(30) Priority: 07.10.2016 EP 16020384
(43) Date of publication of application: 14.08.2019
(73) Proprietor: Boehringer Ingelheim Vetmedica GmbH, 55216 Ingelheim am Rhein (DE)
(72) Inventor: NIEMEYER, Axel, 55216 Ingelheim am Rhein (DE); SCHOEDER, Heinz, 55216 Ingelheim am Rhein (DE); WUERZ, Kai, 55216 Ingelheim am Rhein (DE)
(74) Representative: Von Rohr Patentanwälte Partnerschaft mbB
(86) International application number: PCT/EP2017/025292
(87) International publication number: WO 2018/065115

(56) References cited:
- WO-A1-2010/088514
- US-A1- 2003 224 523
- US-A1- 2007 166 195

## Description

The present invention relates to a method according to claim 1, to an analysis system according to claim 8, and to a computer program product according to claim 15.

Preferably, the present invention deals with analysing and testing a sample, in particular from a human or animal, particularly preferably for analytics and diagnostics, for example with regard to the presence of diseases and/or pathogens and/or for determining blood counts, antibodies, hormones, steroids or the like. Therefore, the present invention is in particular within the field of bioanalytics. A food sample, environmental sample or another sample may optionally also be tested, in particular for environmental analytics or food safety and/or for detecting other substances.

Preferably, by means of the present invention, at least one analyte (target analyte) of a sample can be determined, identified or detected. In particular, the sample can be tested for qualitatively or quantitatively determining at least one analyte, for example in order for it to be possible to detect or identify a disease and/or pathogen.

Within the meaning of the present invention, analytes are in particular nucleic-acid sequences, in particular DNA sequences and/or RNA sequences, and/or proteins, in particular antigens and/or antibodies. In particular, by means of the present invention, nucleic-acid sequences can be determined, identified or detected as analytes of a sample, and/or proteins can be determined, identified or detected as analytes of the sample. More particularly preferably, the present invention deals with systems, devices and other apparatuses for carrying out a nucleic-acid assay for detecting or identifying a nucleic-acid sequence and/or a protein assay for detecting or identifying a protein.

The present invention deals in particular with what are known as point-of-care systems, i.e. in particular with mobile systems, devices and other apparatuses, and deals with methods for carrying out tests on a sample at the sampling site and/or separately or away from a central laboratory or the like. Preferably, point-of-care systems can be operated autonomously or independently of a mains network for supplying electrical power.

US 5,096,669 discloses a point-of-care system for testing a biological sample, in particular a blood sample. The system comprises a single-use cartridge and an analysis device. Once the sample has been received, the cartridge is inserted into the analysis device in order to carry out the test. The cartridge comprises a microfluidic system and a sensor apparatus comprising electrodes, which apparatus is calibrated by means of a calibration liquid and is then used to test the sample.

Furthermore, WO 2006/125767 A1 discloses a point-of-care system for integrated and automated DNA or protein analysis, comprising a single-use cartridge and an analysis device for fully automatically processing and evaluating molecular-diagnostic analyses using the single-use cartridge. The cartridge is designed to receive a sample, in particular blood, and in particular allows cell disruption, PCR and detection of PCR amplification products, which are bonded to capture molecules and provided with a label enzyme, in order for it to be possible to detect bonded PCR amplification products or nucleic sequences as target analytes in what is known as a redox cycling process.

WO 2010/088514 A1 discloses a portable high gain fluorescence detection system. The system comprises a compact, microprocessor-controlled instrument for fluoro-metric assays in liquid samples, the instrument having a floating stage with docking bay for receiving a microfluidic cartridge. The docking bay is suspension-mounted and tilted at an angle relative to the instrument base. A tilt sensor may be used in conjunction with the instrument host controller in order to ensure the proper angle is maintained for improved performance.

US 2007/0166195 A1 discloses an analyzer system, wherein a sample analyzer may be a portable sample analyzer that includes a disposable fluidic cartridge. The sample analyzer may include a level sensor to determine if the sample analyzer is sufficiently level to perform an analysis. If the sample analyzer is not sufficiently level, the sample analyzer may not perform an analysis and/or not provide a result, and in some cases, may provide an error message or error code.

US 2003/0224523 A1 discloses a cartridge arrangement, fluid analyzer arrangement, and methods. A cartridge for analysis of fluid samples usable with an analyzer device is provided. The cartridge includes an arrangement to selectively control fluid flow within the cartridge. The analyzer device may include an output display in order to display the results of the analysis.

In point-of-care systems, it needs to be considered that changeable boundary conditions which are dependent on the installation site may lead to inaccuracies in the test to be carried out. In addition, point-of-care systems are sometimes not operated properly, in particular due to the fact that systems of this type are not used in a laboratory but at the sampling site, where documentation and the like is sometimes not within easy reach, and this can lead to operation errors.

The problem addressed by the present invention is to provide a method, a computer program product and an analysis system in which the reliability, usability and/or ease of operation can be improved.

The above problem is solved by a method according to claim 1, by an analysis system according to claim 8 or by a computer program product according to claim 15. Advantageous developments are the subject of the dependent claims.

The proposed analysis system is preferably designed or provided for testing an in particular biological sample.

The analysis system is in particular portable, mobile and/or is a point-of-care system and/or is or can be operated autonomously, in particular independently from a power network, in particular at the sampling site and/or away from a central laboratory and/or by means of an energy storage unit.

The analysis system preferably comprises an analysis device and a cartridge for testing the sample, the cartridge preferably being designed for receiving the sample. Particularly preferably, the analysis device is designed to receive the cartridge or to connect said cartridge electrically, thermally and/or pneumatically. The analysis device is preferably designed to subsequently carry out the test using the received cartridge. For this purpose, the cartridge can be inserted or loaded into the analysis device, whereupon the analysis device can act on the cartridge in order to carry out the test.

Optionally, the inclination of the analysis device and/or the cartridge is monitored. This means or can ensure that the test can be carried out reliably or can lead to the desired results.

The term "inclination" of the analysis device or the cartridge is preferably understood to mean an inclination relative to an operating position. In other words, the operating position is provided as a reference for the inclination, such that there is no inclination in the operating position and the inclination increases as said analysis device or cartridge moves further away from said operating position by turning or tilting about a non-vertical axis. The inclination can be stated, for example, as an angle and/or in degrees, radians or the like.

In the "operating position", the analysis device is oriented so as to have one side preferably at least substantially horizontal, i.e. perpendicular to the vertical direction. A horizontal orientation of the analysis device is therefore preferably provided when a base of the analysis device, which is arranged at the bottom in a normal operating position and for example comprises device feet, rollers or other support apparatuses or elements, is oriented at least substantially horizontally, i.e. perpendicularly to the direction of action of the gravitational force / vertical direction. With regard to the analysis device, it is assumed that the horizontal orientation preferably always relates to a base plate and/or lower housing face.

The cartridge is preferably a card-like form having a main plane of extension, the main plane of extension, when in the operating position, preferably being oriented in the direction of the gravitational force and/or vertically. Therefore, the term "inclination" is preferably understood to mean a rotational deviation from the operating position, i.e. when the cartridge or the analysis device is pivoted or tilted relative to the operating position.

A main direction of extension or a surface extension direction of the cartridge is in particular at least substantially vertically oriented in the operating position or in the position in which said cartridge is received in the analysis device in the operating position. Here, inclination is preferably understood to mean a deviation from the vertical orientation of the cartridge and/or the horizontal orientation of the analysis device.

Cavities in the cartridge preferably each comprise at least one inlet and one outlet, which, in the operating position, are at different or opposite ends in the vertical direction, thus preferably at an upper and a lower end. In the operating position, the cavities thus preferably extend at least substantially in the direction of or in parallel with the force of gravity at the location of the analysis device.

The sample and optionally other fluids, in particular liquids such as wash buffers or reagents, is/are preferably conveyed using the action of gravity. The cavities may simultaneously contain a gas phase and a liquid phase and/or fluids of different densities. The fact that the gas and/or fluid with lower density rises upwards in the operating position and therefore, depending on the conveying direction, the gas and/or fluid with lower density is or can be removed through the connection that is at the top in the operating position and/or the liquid and/or fluid with higher density is or can be removed through the connection that is at the bottom in the operating position can therefore be utilised in the test, or the cartridge, the analysis device and/or the analysis system is designed to utilise this.

In this regard, it is particularly preferable for the inclination of the analysis device and/or the cartridge to be low, i.e. for the analysis device and/or the cartridge to be operated at least substantially in the operating position with respect to the inclination, while the test is being carried out on the sample. In this regard, monitoring the inclination is advantageous in that the reliability and accuracy of the test can be checked and/or improved.

Preferably, the inclination of the cartridge about an axis that is perpendicular to the vertical (in the operating position) and extends in a plane formed by the card-like cartridge is monitored or measured. Here, an inclination of the flat side of the cartridge is monitored. Alternatively or additionally, the inclination of the cartridge about an axis that is perpendicular to the vertical and extends transversely, in particular perpendicularly, to a plane formed by the card-like cartridge is monitored. Here, turning of the cartridge in which the card remains in the same plane is monitored. However, there are also other solutions. Particularly preferably, the inclination of the cartridge about one or more axes that (each) extend transversely to the vertical and are preferably linearly independent is monitored. The results of the monitoring and/or corresponding measurement results are preferably also referred to as inclination from now on.

The analysis device preferably comprises a tilt sensor and is designed to monitor an inclination of the analysis device and/or the cartridge by means of the tilt sensor.

The analysis device is preferably started, stopped and/or controlled depending on the inclination, in particular by means of a control apparatus of the analysis device which is designed to control the test. For this purpose, the control apparatus can compare measurement results determined by the tilt sensor with a threshold value or can evaluate and/or use said results in other ways in order to control, on this basis, the test and/or actuators to act on the cartridge to carry out the test, or in order to adapt or vary the control.

Preferably, the start of the test is blocked and/or enabled depending on the inclination, in particular depending on or by comparison with a threshold value. Here, the start of the test is preferably blocked if the inclination reaches or exceeds a start threshold value. The control apparatus thus preferably controls the test depending on the inclination and/or when a threshold value, in particular the start threshold value, is exceeded, such that the start of the test is blocked or enabled/effected. The start threshold value can be selected such that the test can be carried out if the inclination is below the start threshold value. By means of the start threshold value, the probability of a successful test can therefore be improved.

Alternatively or additionally, it is provided that the test is or can be interrupted depending on the inclination, preferably if the inclination reaches or exceeds an interruption threshold value. For this purpose, the control apparatus is set up to compare, or the analysis device is set up to compare, by means of the control apparatus, the inclination or the measurement results determined by the tilt sensor with a threshold value, in particular an interruption threshold value, and to interrupt the test depending on the result of this comparison and/or to detect, signal and/or store an error.

The use of the interruption threshold value is advantageous in that the test can be interrupted if a successful test can no longer be expected or if continuing with the test could prove detrimental, for example if portions of the sample or other substances could escape through supply and/or exhaust ventilation openings.

The interruption threshold value preferably differs from the start threshold value. Particularly preferably, the interruption threshold value corresponds to a greater inclination than the start threshold value.

Alternatively or additionally, an error is detected, signalled and/or stored depending on the inclination. This can be carried out by a threshold-value comparison of the measurement results from the tilt sensor with a threshold value that may correspond to or differ from the start threshold value or the interruption threshold value. The error can be stored, in particular stored temporarily, in order to be taken into account, displayed and/or output in a subsequent evaluation of the test. The error can, however, also be directly output and/or signalled.

In particular, if an inclination is detected that is below an interruption threshold value, but above a critical value, which can be the start threshold value or another threshold value, an error can be stored and optionally also output. However, in this case it is provided that the test is continued as long as the inclination does not exceed the interruption threshold value.

Alternatively or additionally, a plurality of threshold values below an interruption value can be provided and an error can be or is detected, signaled and/or stored each time the inclination exceeds and/or falls below one of said threshold values.

The stored error preferably comprises error information, for example the threshold value that has been exceeded and/or at which step of the test procedure or test sequence the error occurred. In particular, the stored error or errors can be taken into account when the test is evaluated, for example by changing the evaluation procedure accordingly in order to reduce or compensate for the error/errors.

Alternatively or additionally, measurement results from the tilt sensor can be stored every time the inclination (significantly) changes and/or at certain points during the test, for example at constant time intervals or when the next test step in the test sequence is started. Advantageously, these measurement results can be or are taken into account when evaluating the test.

The threshold value, start threshold value and/or the interruption threshold value can preferably be determined, set or changed, preferably depending on the cartridge or batch of cartridges using which the test is intended to be carried out, and/or on a cartridge identifier of the cartridge, and/or on control information or calibration information for carrying out the test using the cartridge.

Optionally, the test, in particular the sequence thereof, is controlled depending on the inclination. It is thus possible for the test sequence or the control of actuators for carrying out the test on the sample received in the cartridge to be influenced by the determined inclination. In particular, the inclination is compared with a/the threshold value, interruption threshold value and/or start threshold value for this purpose, and this forms the basis for the control. However, it is also possible for there to be a plurality of or several threshold values, an approval, a function or the like and, as a result of this or in another way, the test or control is carried out continuously or discontinuously depending on the inclination.

For example, it is possible for the analysis device and/or the cartridge to be inclined after the start of the test. It is often not practical to stop the test in such a situation, provided that a (partial) result is still possible from the test being carried out in this position. This is the case in particular if the inclination exceeds the start threshold value after the start, but the interruption threshold value has not yet been reached. It is preferable here for the inclination to be taken into account when controlling the test.

In particular, the control can be adapted and/or the test can be controlled depending on the inclination such that deviations caused by the inclination can be reduced or compensated for. A pump drive can for example be actuated in a different manner or for longer, or other actuators of the analysis device can be controlled depending on the inclination.

The threshold value, in particular the start threshold value and/or the interruption threshold value, is preferably determined, set and/or changed. Here, said value is preferably determined, set and/or changed or adapted taking into account the cartridge, the cartridge type from several different supported cartridges, a cartridge identifier for identifying a specific cartridge or batch of cartridges, and taking into account the analysis device, the test and/or control information for carrying out, in particular controlling, the test.

Therefore, it is possible for the analysis device to support different tests using the same cartridge or different cartridges, one of these different cartridges and/or tests being selected and the control being configured for carrying out this selected test depending on the inclination, and/or the start threshold value, the interruption threshold value or another threshold value being determined for comparison with the inclination depending on the selected test.

The threshold value, start threshold value and/or interruption threshold value can be stored or temporarily stored in a memory of the control apparatus, for example, before the test is carried out. This makes it possible for the control apparatus to compare, in each case, the current value for the inclination and/or the measured value from the tilt sensor corresponding thereto with the respective threshold values before or during the test, or to evaluate the inclination in a different manner and, preferably in the manner described, to cause the test and/or the analysis device to be controlled.

For orientation in relation to the inclination, i.e. to reach the operating position, the analysis device preferably comprises one or more support elements, which are designed to orient the analysis device and the cartridge received in the analysis device. In particular, these are height-adjustable feet, by means of which the inclination of the analysis device and therefore also of the cartridge can be adjusted.

In principle, the analysis device may however also comprise other support apparatuses designed to carry the analysis device, i.e. to establish direct contact between the analysis device and a surface underneath in order for the analysis device to be put down or placed.

Preferably, the analysis device comprises in any case two height-adjustable support elements. Together with at least one additional, optionally fixed or likewise height-adjustable support element, the height-adjustable support elements can allow orientation about different axes oriented transversely to the vertical and/or make it possible for the operating position to be reached.

In one embodiment of the analysis device, it is preferable for the analysis device to support automatic orientation, such that the analysis device automatically moves into its operating position. For this purpose, it may be provided that the analysis device measures the inclination using the tilt sensor and compensates for, in particular controls, the inclination by orienting the analysis device.

For this purpose, one, preferably two or more, support elements of the analysis device can be movable relative to a housing of the analysis device, the support element(s) in particular being automatically adjustable, in particular being retractable into the housing or extendable out of the housing, in order to tilt and/or incline the analysis device relative to a surface underneath on which the analysis device is placed. By means of the support elements, the analysis device can then compensate for the inclination in order to reach the operating position.

The support apparatus(es) can be controlled here such that automatic inclination of the analysis device relative to the surface underneath is supported. As an alternative or in addition to retractable and extendable support elements, in this regard other mechanics, such as joints and the like, come under consideration in order to allow automatic or manual inclination of the analysis device.

According to one aspect of the present invention, which can be implemented independently, the analysis device comprises a light strip as a display apparatus, by means of which the analysis device signals or can signal an operating status and/or a requirement.

A "light strip" is preferably an apparatus for the controllable output of light, i.e. electromagnetic waves at wavelengths in the range of from 380 nm to 780 nm. Preferably, the light strip is designed to generate light that is visible to the human eye and is of different colours, including secondary colours or mixed colours. The light strip may for example be designed to emit blue, yellow, green, red and/or white light. Furthermore, the light strip preferably has a strip-like, elongate, in particular self-contained or closed, shape.

Using the light strip, operating states or requirements of the analysis device, in particular the need to be cleaned and/or the need for maintenance and/or an error and/or a wireless connection status and/or a test process, is or can be signalled or output. The operating states can be signalled by light being emitted that is preferably colour-coded and/or coded by flashing. In particular, a status of a wireless data connection to an operating instrument, a status of the energy storage means, and/or a status relating to the test can be signalled.

Optionally, it is provided that or the analysis device is designed such that the light strip signals the inclination. Here, the light strip can function as an electronic spirit level. If the inclination is greater than a threshold value, in particular greater than the interruption threshold value and/or the start threshold value, this can be represented by an asymmetrical or uneven or non-uniform display on the light strip. Alternatively or additionally, an inclination that is less than the threshold value, in particular less than the interruption threshold value and/or the start threshold value, can be represented by a symmetrical or even or uniform display on the light strip.

Another aspect of the present invention, which can also be implemented independently, relates to a computer program product comprising program code means which, when executed, cause the following steps of the method to be implemented: controlling an analysis device (200), the analysis device (200) being designed for testing an in particular biological sample (P), it being possible for the sample (P) to be received in a cartridge (100), and the analysis device (200) being designed for receiving the cartridge (100) and subsequently carrying out the test using the received cartridge (100), and
signaling an operating status and/or a requirement by means of a light strip (209A) as a display apparatus (209) of the analysis device (200), the light strip (209A) being provided at least substantially preferably and/or all round on the analysis device (200).

The program code means may be provided on a computer-readable storage medium. In particular, said means are stored in a database and/or can be transmitted to the analysis device and/or can be received by the analysis device and executed by the analysis device, such that the analysis device facilitates, supports or implements
the described method(s). The computer program product preferably is a non-transitory computer-readable media.

The term "analysis device" is preferably understood to mean an instrument which is in particular mobile and/or can be used on site, and/or which is designed to chemically, biologically and/or physically test and/or analyse a sample or a component thereof, preferably in and/or by means of a cartridge. In particular, the analysis device controls the pretreatment and/or testing of the sample in the cartridge. For this purpose, the analysis device can act on the cartridge, in particular such that the sample is conveyed, temperature-controlled and/or measured in the cartridge.

The term "cartridge" is preferably understood to mean a structural apparatus or unit designed to receive, to store, to physically, chemically and/or biologically treat and/or prepare and/or to measure a sample, preferably in order to make it possible to detect, identify or determine at least one analyte, in particular a protein and/or a nucleic-acid sequence, of the sample.

A cartridge within the meaning of the present invention preferably comprises a fluid system having a plurality of channels, cavities and/or valves for controlling the flow through the channels and/or cavities.

In particular, within the meaning of the present invention, a cartridge is designed to be at least substantially planar, flat and/or card-like, in particular is designed as a (micro)fluidic card and/or is designed as a main body or container that can preferably be closed and/or said cartridge can be inserted and/or plugged into a proposed analysis device when it contains the sample.

The term "test" as used herein preferably means a test procedure and/or performing an assay, in particular one, several or all steps for performing an assay to determine one or more analytes of a sample. The steps are preferably realized by or within the analysis system, analysis device and/or cartridge.

An "assay" according to the present invention is preferably an investigative procedure for qualitatively and/or quantitatively measuring, detecting and/or identifying the presence, amount, and/or functional activity of a target entity or analyte of the sample. The analyte can, e.g., be a drug, a biological, chemical and/or biochemical substance, and/or a cell in an organism or organic sample. In particular, the analyte can be a molecule, a nucleic-acid sequence, a DNA, an RNA and/or a protein.

Preferably, the assay according to the present invention is a nucleic-acid assay for detecting or identifying a nucleic-acid sequence and/or a protein assay for detecting or identifying a protein.

An assay, test or test procedure according to the present invention accordingly preferably covers at least one of: controlling actuators of the analysis device like a pump drive, temperature control apparatus, and valve actuators; acting on the cartridge or sample; treating the sample; preparing the sample; performing one or more mixing processes and/or reactions with the sample; conveying the sample; and measuring one or more properties of the sample, particularly with the sensor apparatus of the cartridge.

An assay, test or test procedure according to the present invention preferably starts or begins with the analysis device acting on and/or controlling processes on the cartridge and/or the sample. In particular, a test starts or begins with actuators acting on the cartridge. For example, a test can start with conveying the sample within the cartridge.

Methods and/or steps performed before insertion or receiving of the cartridge in-to/by the analysis device and/or before conveying, treating and/or preparing the sample within said cartridge are preferably not part of an assay, test or test procedure according to the present invention.

The "control information", thus, preferably is configured to carry out such an assay, test or test procedure or to enable the analysis system or the analysis device to carry out such an assay, test or test procedure. Preferably, said control information is configured to control or to define a control sequence or to be used by the analysis device to carry out said assay, test or test procedure. A "control information", thus, preferably has instructions being configured for controlling the assay, test or test procedure. In particular, the control information is configured to control an assay, test or test procedure by defining steps or parameters of steps including controlling and/or feedback controlling actuators like the pump drive, the temperature control apparatuses and valve actuators.

The term "operating instrument" is preferably understood to mean an apparatus by means of which the analysis device can be controlled, control information can be transmitted to the analysis device, and/or measurement results can be received from the analysis device and/or measurement results can be evaluated. Preferably, the operating instrument is or forms a user interface for controlling the test and/or the evaluation or outputting of measurement results.

The operating instrument can alternatively be called operator control instrument. The operating instrument preferably is configured to be operated by an operator (user) for controlling, in particular of the analysis device, the test and/or the evaluation. Thus, the operating instrument is or comprises a user interface for input of commands and transfer of pieces of control information to the analysis device.

The operating instrument preferably comprises an input apparatus for controlling the analysis device, for controlling data transmission and/or for controlling the evaluation of measurement results. Alternatively or additionally, the operating instrument comprises an output apparatus for outputting, in particular displaying, information, in particular status information, operating elements and/or results. The operating instrument preferably comprises a processor, microcontroller and/or memory for executing a computer program product for data transmission, for control and/or for evaluating measurement results.

Particularly preferably, the operating instrument is a mobile terminal device, in particular for a radio and/or mobile network, such as a smartphone, tablet computer, mobile telephone or the like. The operating instrument can preferably be operated independently from a power network, using a power storage means, in particular a (rechargeable) battery, and in a mobile manner, autonomously of and/or independently from further components of the analysis system, in particular the analysis device. The operating instrument preferably comprises one or more interfaces for wireless data communications, in particular a WPAN communication interface, a WLAN communication interface, a near-field communication interface, an optical communication interface such as a camera, and/or a mobile radio interface.

The above-mentioned aspects and features of the present invention and the aspects and features of the present invention that will become apparent from the claims and the following description can in principle be implemented independently from one another, but also in any combination or order.

Other aspects, advantages, features and properties of the present invention will become apparent from the claims and the following description of a preferred embodiment with reference to the drawings, in which:
- Fig. 1: is a schematic view of a proposed analysis system or analysis device comprising a proposed cartridge received therein;
- Fig. 2: is a schematic view of the cartridge;
- Fig. 3: is a schematic view of the proposed analysis system; and
- Fig. 4: shows the analysis device in different inclination positions.

In the Figures, which are only schematic and sometimes not to scale, the same reference signs are used for the same or similar parts and components, corresponding or comparable properties and advantages being achieved even if these are not repeatedly described.

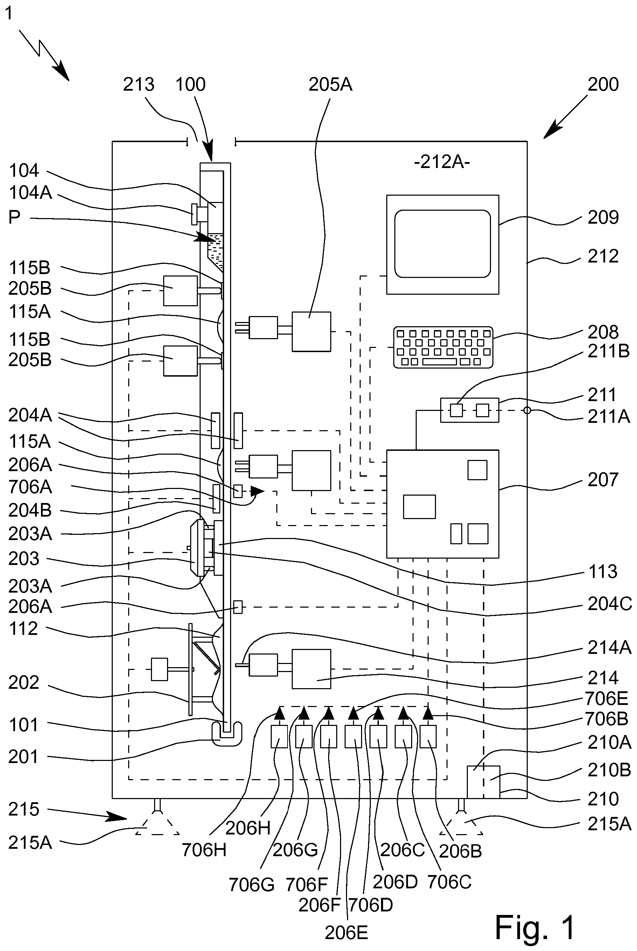

Fig. 1 is a highly schematic view of a proposed analysis system 1 and analysis device 200 for testing an in particular biological sample P, preferably by means of or in an apparatus or cartridge 100.

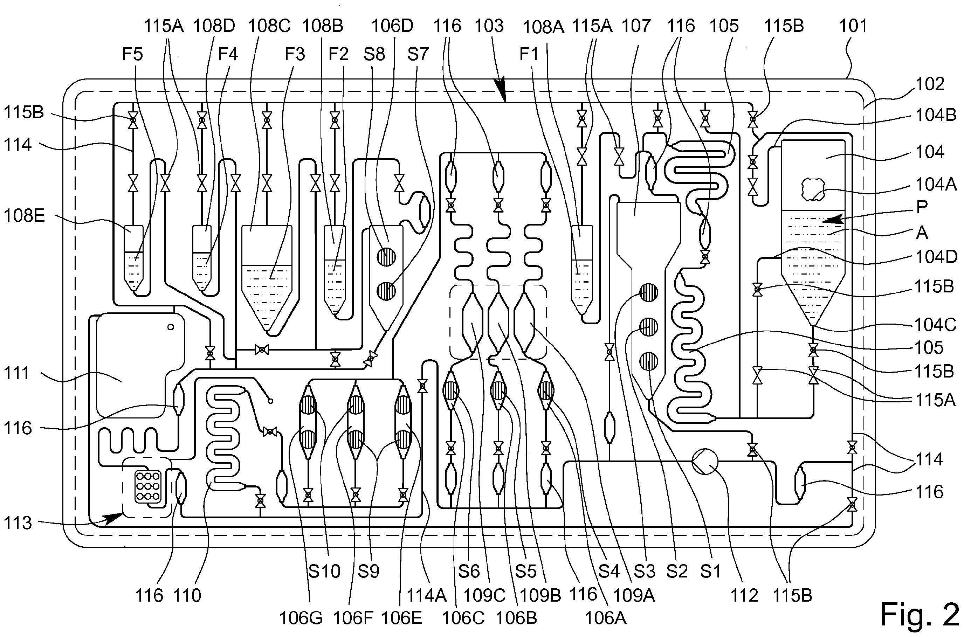

Fig. 2 is a schematic view of a preferred embodiment of the proposed apparatus or cartridge 100 for testing the sample P. The apparatus or cartridge 100 in particular forms a handheld unit, and in the following is merely referred to as a cartridge.

The term "sample" is preferably understood to mean the sample material to be tested, which is in particular taken from a human or animal. In particular, within the meaning of the present invention, a sample is a fluid, such as saliva, blood, urine or another liquid, preferably from a human or animal, or a component thereof. Within the meaning of the present invention, a sample may be pretreated or prepared if necessary, or may come directly from a human or animal or the like, for example. A food sample, environmental sample or another sample may optionally also be tested, in particular for environmental analytics, food safety and/or for detecting other substances, preferably natural substances, but also biological or chemical warfare agents, poisons or the like.

Preferably, the analysis system 1 and/or analysis device 200 controls the testing of the sample P in particular in or on the cartridge 100 and/or is used to evaluate the testing and/or to collect, to process and/or to store measurement results from the test.

The analysis system 1 preferably comprises one or more cartridges 100 for receiving the sample P. The analysis system 1 preferably comprises the analysis device 200 for receiving the cartridge 100 and subsequently carrying out the test using the received cartridge 100.

By means of the proposed analysis system 1, analysis device 200 and/or cartridge 100 and/or using the proposed method for testing the sample P, preferably an analyte A of the sample P, in particular a (certain) nucleic-acid sequence and/or a (certain) protein, or particularly preferably a plurality of analytes A of the sample P, can be determined, identified or detected. Said analytes A are in particular detected, identified and/or measured not only qualitatively, but particularly preferably also quantitatively.

Therefore, the sample P can in particular be tested for qualitatively or quantitatively determining at least one analyte A, for example in order for it to be possible to detect a disease and/or pathogen or to determine other values, which are important for diagnostics, for example.

Particularly preferably, a molecular-biological test is made possible by means of the analysis system 1 and/or analysis device 200 and/or by means of the cartridge 100.

Particularly preferably, a nucleic-acid assay for detecting a nucleic-acid sequence, in particular a DNA sequence and/or RNA sequence, and/or a protein assay for detecting a protein, in particular an antigen and/or antibody, are made possible or are carried out.

Preferably, the sample P or individual components of the sample P or analyte A can be amplified if necessary, in particular by means of PCR, and tested, identified or detected in the analysis system 1, analysis device 200 and/or in the cartridge 100, and/or for the purpose of carrying out the nucleic-acid assay. Preferably, amplification products of the analyte A or analytes A are thus produced.

In the following, further details are first given on a preferred construction of the cartridge 100, with features of the cartridge 100 preferably also directly representing features of the analysis system 1, in particular even without any further explicit explanation.

The cartridge 100 is preferably at least substantially planar, flat, plate-shaped and/or card-like.

The cartridge 100 preferably comprises an in particular at least substantially planar, flat, plate-shaped and/or card-like main body or support 101, the main body or support 101 in particular being made of and/or injection-moulded from plastics material, particularly preferably polypropylene.

The cartridge 100 preferably comprises at least one film or cover 102 for covering the main body 101 and/or cavities and/or channels formed therein at least in part, in particular on the front, and/or for forming valves or the like, as shown by dashed lines in Fig. 2.

The analysis system 1 or cartridge 100 or the main body 101 thereof, in particular together with the cover 102, preferably forms and/or comprises a fluidic system 103, referred to in the following as the fluid system 103.

The cartridge 100, the main body 101 and/or the fluid system 103 are preferably at least substantially vertically oriented in the operating position and/or during the test, in particular in the analysis device 200, as shown schematically in Fig. 1. In particular, the main plane or surface extension of the cartridge 100 thus extends at least substantially vertically in the operating position.

The cartridge 100 and/or the fluid system 103 preferably comprises a plurality of cavities, in particular at least one receiving cavity 104, at least one metering cavity 105, at least one intermediate cavity 106A-G, at least one mixing cavity 107, at least one storage cavity 108, at least one reaction cavity 109A-C, at least one intermediate temperature-control cavity 110 and/or at least one collection cavity 111, as shown in Fig. 1 and Fig. 2.

The cartridge 100 and/or the fluid system 103 also preferably comprises at least one pump apparatus 112 and/or at least one sensor arrangement or sensor apparatus 113.

Some, most or all of the cavities are preferably formed by chambers and/or channels or other depressions in the cartridge 100 and/or the main body 101, and particularly preferably are covered or closed by the cover 102. However, other structural solutions are also possible.

In the example shown, the cartridge 100 or the fluid system 103 preferably comprises two metering cavities 105, a plurality of intermediate cavities 106A to 106G, a plurality of storage cavities 108A to 108E and/or a plurality of reaction cavities 109A-C, which can preferably be loaded separately from one another, in particular a first reaction cavity 109A, a second reaction cavity 109B and an optional third reaction cavity 109C, as can be seen in Fig. 2.

The reaction cavity/cavities 109A-C is/are used in particular to carry out an amplification reaction, in particular PCR, or several, preferably different, amplification reactions, in particular PCRs. It is preferable to carry out several, preferably different, PCRs, i.e. PCRs having different primer combinations or primer pairs, in parallel and/or independently and/or in different reaction cavities 109A-C.

To carry out the nucleic-acid assay, preferably nucleic-acid sequences, as analytes A of the sample P, are amplified in the reaction cavity/cavities 109A-C by means of an amplification reaction, in particular in order to produce amplification products for the subsequent detection in the sensor arrangement or sensor apparatus 113.

Within the meaning of the present invention, amplification reactions are in particular molecular-biological reactions in which an analyte A, in particular a nucleic-acid sequence, is amplified/copied and/or in which amplification products, in particular nucleic-acid products, of an analyte A are produced. Particularly preferably, PCRs are amplification reactions within the meaning of the present invention.

"PCR" stands for polymerase chain reaction and is a molecular-biological method by means of which certain analytes A, in particular portions of RNA or RNA sequences or DNA or DNA sequences, of a sample P are amplified, preferably in several cycles, using polymerases or enzymes, in particular in order to then test and/or detect the amplification products or nucleic-acid products. If RNA is intended to be tested and/or amplified, before the PCR is carried out, a cDNA is produced starting from the RNA, in particular using reverse transcriptase. The cDNA is used as a template for the subsequent PCR.

Preferably, during a PCR, a sample P is first denatured by the addition of heat in order to separate the strands of DNA or cDNA. Preferably, primers or nucleotides are then deposited on the separated single strands of DNA or cDNA, and a desired DNA or cDNA sequence is replicated by means of polymerase and/or the missing strand is replaced by means of polymerase. This process is preferably repeated in a plurality of cycles until the desired quantity of the DNA or cDNA sequence is available.

For the PCR, marker primers are preferably used, i.e. primers which (additionally) produce a marker or a label, in particular biotin, on the amplified analyte A or amplification product. This allows or facilitates detection. Preferably, the primers used are biotinylated and/or comprise or form in particular covalently bonded biotin as the label.

The amplification products and/or other portions of the sample P produced in the one or more reaction cavities 109A-C can be conducted or fed to the connected sensor arrangement or sensor apparatus 113, in particular by means of the pump apparatus 112.

The sensor apparatus 113 is used in particular for detecting, particularly preferably qualitatively and/or quantitatively determining, the analyte A or analytes A of the sample P, in this case particularly preferably the nucleic-acid sequences and/or proteins as the analytes A. Alternatively or additionally, however, other values may also be collected or determined.

As already explained at the outset, in particular nucleic-acid sequences, preferably DNA sequences and/or RNA sequences, and/or proteins, in particular antigens and/or antibodies, are preferably qualitatively and/or quantitatively determined as analytes A of the sample P. In the following, however, a distinction is not made between nucleic-acid sequences and proteins, or between the nucleic-acid assay for detecting nucleic-acid sequences and the protein assay for detecting proteins.

In particular, the pump apparatus 112 comprises or forms a tube-like or bead-like raised portion, in particular by means of the film or cover 102, particularly preferably on the back of the cartridge 100, as shown schematically in Fig. 1.

The cartridge 100, the main body 101 and/or the fluid system 103 preferably comprise a plurality of channels 114 and/or valves 115A, 115B, as shown in Fig. 2.

By means of the channels 114 and/or valves 115A, 115B, the cavities 104 to 111, the pump apparatus 112 and/or the sensor arrangement and/or sensor apparatus 113 can be temporarily and/or permanently fluidically interconnected and/or fluidically separated from one another, as required and/or optionally or selectively, in particular such that they are controlled by the analysis system 1 or the analysis device 200.

The cavities 104 to 111 are preferably each fluidically linked or interconnected by a plurality of channels 114. Particularly preferably, each cavity is linked or connected by at least two associated channels 114, in order to make it possible for fluid to fill, flow through and/or drain from the respective cavities as required.

The fluid transport or the fluid system 103 is preferably not based on capillary forces, or is not exclusively based on said forces, but in particular is essentially based on the effects of gravity and/or pumping forces and/or compressive forces and/or suction forces that arise, which are particularly preferably generated by the pump or pump apparatus 112. In this case, the flows of fluid or the fluid transport and the metering are controlled by accordingly opening and closing the valves 115A, 115B and/or by accordingly operating the pump or pump apparatus 112, in particular by means of a pump drive 202 of the analysis device 200.

Preferably, each of the cavities 104 to 110 has an inlet at the top and an outlet at the bottom in the operating position. Therefore, if required, only liquid from the respective cavities can be removed via the outlet.

In the operating position, the liquids from the respective cavities are preferably removed, in particular drawn out, via the outlet that is at the bottom in each case, it preferably being possible for gas or air to flow and/or be pumped into the respective cavities via the inlet that is in particular at the top. In particular, relevant vacuums in the cavities can thus be prevented or at least minimised when conveying the liquids.

In particular, the cavities, particularly preferably the storage cavity/cavities 108, the mixing cavity 107 and/or the receiving cavity 104, are each dimensioned and/or oriented in the normal operating position such that, when said cavities are filled with liquid, bubbles of gas or air that may potentially form rise upwards in the operating position, such that the liquid collects above the outlet without bubbles. However, other solutions are also possible here.

The receiving cavity 104 preferably comprises a connection 104A for introducing the sample P. In particular, the sample P may for example be introduced into the receiving cavity 104 and/or cartridge 100 via the connection 104A by means of a pipette, syringe or other instrument.

The receiving cavity 104 preferably comprises an inlet 104B, an outlet 104C and an optional intermediate connection 104D, it preferably being possible for the sample P or a portion thereof to be removed and/or conveyed further via the outlet 104C and/or the optional intermediate connection 104D. Gas, air or another fluid can flow in and/or be pumped in via the inlet 104B, as already explained.

Preferably, the sample P or a portion thereof can be removed, optionally and/or depending on the assay to be carried out, via the outlet 104C or the optional intermediate connection 104D of the receiving cavity 104. In particular, a supernatant of the sample P, such as blood plasma or blood serum, can be conducted away or removed via the optional intermediate connection 104D, in particular for carrying out the protein assay.

Preferably, at least one valve 115A, 115B is assigned to each cavity, the pump apparatus 112 and/or the sensor apparatus 113 and/or is arranged upstream of the respective inlets and/or downstream of the respective outlets.

Preferably, the cavities 104 to 111 or sequences of cavities 104 to 111, through which fluid flows in series or in succession for example, can be selectively released and/or fluid can selectively flow therethrough by the assigned valves 115A, 115B being actuated, and/or said cavities can be fluidically connected to the fluid system 103 and/or to other cavities.

In particular, the valves 115A, 115B are formed by the main body 101 and the film or cover 102 and/or are formed in another manner, for example by additional layers, depressions or the like.

Particularly preferably, one or more valves 115A are provided which are preferably tightly closed initially or in the storage state, particularly preferably in order to seal liquids or liquid reagents F, located in the storage cavities 108, and/or the fluid system 103 from the open receiving cavity 104 in a storage-stable manner.

Preferably, an initially closed valve 115A is arranged upstream and downstream of each storage cavity 108. Said valves are preferably only opened, in particular automatically, when the cartridge 100 is actually being used and/or while inserting the cartridge 100 into the analysis device 200 and/or for carrying out the assay.

A plurality of valves 115A, in particular three valves in this case, are preferably assigned to the receiving cavity 104, in particular if the intermediate connection 104D is provided in addition to the inlet 104B and the outlet 104C. Depending on the use, in addition to the valve 115A on the inlet 104B, then preferably only the valve 115A either at the outlet 104C or at the intermediate connection 104D is opened.

The valves 115A assigned to the receiving cavity 104 seal the fluid system 103 and/or the cartridge 100 in particular fluidically and/or in a gas-tight manner until the sample P is inserted and the receiving cavity 104 or a connection 104A of the receiving cavity 104 is closed.

As an alternative or in addition to the valves 115A (which are initially closed), one or more valves 115B are preferably provided which are not closed in a storage-stable manner and/or which are open initially and/or which can be closed by actuation. These valves are used in particular to control the flows of fluid during the test.

The cartridge 100 is preferably designed as a microfluidic card and/or the fluid system 103 is preferably designed as a microfluidic system. In the present invention, the term "microfluidic" is preferably understood to mean that the respective volumes of individual cavities, some of the cavities or all of the cavities 104 to 111 and/or channels 114 are, separately or cumulatively, less than 5 ml or 2 ml, particularly preferably less than 1 ml or 800 µl, in particular less than 600 µl or 300 µl, more particularly preferably less than 200 µl or 100 µl.

Particularly preferably, a sample P having a maximum volume of 5 ml, 2 ml or 1 ml can be introduced into the cartridge 100 and/or the fluid system 103, in particular the receiving cavity 104.

Reagents and liquids which are preferably introduced or provided before the test in liquid form as liquids or liquid reagents F and/or in dry form as dry reagents S are required for testing the sample P, as shown in the schematic view according to Fig. 2 by reference signs F1 to F5 and S1 to S10.

Furthermore, other liquids F, in particular in the form of a wash buffer, solvent for dry reagents S and/or a substrate, for example in order to form detection molecules and/or a redox system, are also preferably required for the test, the detection process and/or for other purposes, and are in particular provided in the cartridge 100, i.e. are likewise introduced before use, in particular before delivery. At some points in the following, a distinction is not made between liquid reagents and other liquids, and therefore the respective explanations are accordingly also mutually applicable.

The analysis system 1 or the cartridge 100 preferably contains all the reagents and liquids required for pretreating the sample P and/or for carrying out the test or assay, in particular for carrying out one or more amplification reactions or PCRs, and therefore, particularly preferably, it is only necessary to receive the optionally pretreated sample P.

The cartridge 100 or the fluid system 103 preferably comprises a bypass 114A that can optionally be used, in order for it to be possible, if necessary, to conduct or convey the sample P or components thereof past the reaction cavities 109A-C and/or, by bypassing the optional intermediate temperature-control cavity 110, also directly to the sensor apparatus 113.

The cartridge 100, the fluid system 103 and/or the channels 114 preferably comprise sensor portions 116 or other apparatuses for detecting liquid fronts and/or flows of fluid.

It is noted that various components, such as the channels 114, the valves 115A, 115B, in particular the valves 115A that are initially closed and the valves 115B that are initially open, and the sensor portions 116 in Fig. 2 are, for reasons of clarity, only labelled in some cases, but the same symbols are used in Fig. 2 for each of these components.

The collection cavity 111 is preferably used for receiving excess or used reagents and liquids and volumes of the sample, and/or for providing gas or air in order to empty individual cavities and/or channels.

In particular, the collection cavity 111 can optionally be connected to individual cavities and channels or other apparatuses fluidically in order to remove reagents and liquids from said cavities, channels or other apparatuses and/or to replace said reagents and liquids with gas or air. The collection cavity 111 is preferably given appropriate large dimensions.

Once the sample P has been introduced into the receiving cavity 104 and the connection 104A has been closed, the cartridge 100 can be inserted into and/or received in the proposed analysis device 200 in order to test the sample P, as shown in Fig. 1. Alternatively, the sample P could also be fed in later.

Fig. 1 shows the analysis system 1 in a ready-to-use state for carrying out a test or assay on the sample P received in the cartridge 100, and/or in the operating position. In this state, the cartridge 100 is therefore linked to, received by and/or inserted into the analysis device 200.

In the following, some features and aspects of the analysis device 200 are first explained in greater detail, in particular on the basis of Fig. 1. The features and aspects relating to said device are preferably also directly features and aspects of the proposed analysis system 1, in particular even without any further explicit explanation.

The analysis system 1 or analysis device 200 preferably comprises a mount or receptacle 201 for mounting and/or receiving the cartridge 100.

Preferably, the cartridge 100 is fluidically, in particular hydraulically, separated or isolated from the analysis device 200. In particular, the cartridge 100 forms a preferably independent and in particular closed or sealed fluidic or hydraulic system 103 for the sample P and the reagents and other liquids. In this way, the analysis device 200 does not come into direct contact with the sample P and can in particular be reused for another test without being disinfected and/or cleaned first.

It is however provided that the analysis device 200 can be connected or coupled mechanically, electrically, thermally and/or pneumatically to the cartridge 100.

In particular, the analysis device 200 is designed to have a mechanical effect, in particular for actuating the pump apparatus 112 and/or the valves 115A, 115B, and/or to have a thermal effect, in particular for temperature-controlling the reaction cavity/cavities 109A-C and/or the intermediate temperature-control cavity 110.

In addition, the analysis device 200 can preferably be pneumatically connected to the cartridge 100, in particular in order to actuate individual apparatuses, and/or can be electrically connected to the cartridge 100, in particular in order to collect and/or transmit measured values or measurement results 713, for example from the sensor apparatus 113 and/or sensor portions 116.

The analysis system 1 or analysis device 200 preferably comprises a pump drive 202, the pump drive 202 in particular being designed for mechanically actuating the pump apparatus 112.

Preferably, a head of the pump drive 202 can be rotated in order to rotationally axially depress the preferably bead-like raised portion of the pump apparatus 112. Particularly preferably, the pump drive 202 and pump apparatus 112 together form a pump, in particular in the manner of a hose pump or peristaltic pump and/or a metering pump, for the fluid system 103 and/or the cartridge 100.

Particularly preferably, the pump is constructed as described in DE 10 2011 015 184 B4. However, other structural solutions are also possible.

Preferably, the capacity and/or discharge rate of the pump can be controlled and/or the conveying direction of the pump and/or pump drive 202 can be switched. Preferably, fluid can thus be pumped forwards or backwards as desired.

The analysis system 1 or analysis device 200 preferably comprises a connection apparatus 203 for in particular electrically and/or thermally connecting the cartridge 100 and/or the sensor arrangement or sensor apparatus 113.

As shown in Fig. 1, the connection apparatus 203 preferably comprises a plurality of electrical contact elements 203A, the cartridge 100, in particular the sensor arrangement or sensor apparatus 113, preferably being electrically connected or connectable to the analysis device 200 by the contact elements 203A.

The analysis system 1 or analysis device 200 preferably comprises one or more temperature-control apparatuses for temperature-controlling the cartridge 100 and/or having a thermal effect on the cartridge 100, in particular for heating and/or cooling, the temperature-control apparatus(es) (each) preferably comprising or being formed by a heating resistor or a Peltier element.

Individual temperature-control apparatuses, some of these apparatuses or all of these apparatuses can preferably be positioned against or abutted on the cartridge 100, the main body 101, the cover 102, the sensor arrangement, sensor apparatus 113 and/or individual cavities and/or can be thermally coupled thereto and/or can be integrated therein and/or in particular can be operated or controlled electrically by the analysis device 200. In the example shown, in particular the temperature-control apparatuses 204A-C are provided.

Preferably, the temperature-control apparatus, referred to in the following as the reaction temperature-control apparatus 204A, is assigned to one of the reaction cavities 109A-C or to a plurality of reaction cavities 109A-C, in particular in order for it to be possible to carry out one or more amplification reactions therein.

The reaction cavities 109A-C are preferably temperature-controlled simultaneously and/or uniformly, in particular by means of one common reaction temperature-control apparatus 204A or two reaction temperature-control apparatuses 204A.

More particularly preferably, the reaction cavity/cavities 109A-C can be temperature-controlled from two different sides and/or by means of two or the reaction temperature-control apparatuses 204A that are preferably arranged on opposite sides.

Alternatively, each reaction cavity 109A-C can be temperature-controlled independently and/or individually.

The temperature-control apparatus, referred to in the following as the intermediate temperature-control apparatus 204B, is preferably assigned to the intermediate temperature-control cavity 110 and/or is designed to (actively) temperature-control or heat the intermediate temperature-control cavity 110 and/or a fluid located therein, in particular the amplification products, preferably to a preheat temperature.

The intermediate temperature-control cavity 110 and/or intermediate temperature-control apparatus 204B is preferably arranged upstream of or (immediately) before the sensor arrangement or sensor apparatus 113, in particular in order for it to be possible to temperature-control or preheat, in a desired manner, fluids to be fed to the sensor arrangement or sensor apparatus 113, in particular analytes A and/or amplification products, particularly preferably immediately before said fluids are fed.

Particularly preferably, the intermediate temperature-control cavity 110 or intermediate temperature-control apparatus 204B is designed or provided to denature the sample P or analytes A and/or the amplification products produced, and/or to divide any double-stranded analytes A or amplification products into single strands and/or to counteract premature bonding or hybridising of the amplification products, in particular by the addition of heat.

Preferably, the analysis system 1, analysis device 200 and/or the cartridge 100 and/or one or each temperature-control apparatus comprise/comprises a temperature detector and/or temperature sensor (not shown), in particular in order to make it possible to control and/or regulate temperature.

One or more temperature sensors may for example be assigned to the sensor portions 116 and/or to individual channel portions or cavities, i.e. may be thermally coupled thereto.

The temperature-control apparatus 204C, referred to in the following as the sensor temperature-control apparatus 204C, is in particular assigned to the sensor apparatus 113 and/or is designed to (actively) temperature-control or heat fluids located in or on the sensor arrangement or sensor apparatus 113, in particular analytes A and/or amplification products, reagents or the like, in a desired manner, preferably to a hybridisation temperature.

The analysis system 1 or analysis device 200 preferably comprises one or more valve actuators 205A, B for actuating the valves 115A, 115B. Particularly preferably, different (types or groups of) valve actuators 205A and 205B are provided which are assigned to the different (types or groups of) valves 115A and 115B for actuating each of said valves, respectively.

The analysis system 1 or analysis device 200 preferably comprises a control apparatus 207 for controlling the sequence of a test or assay and/or for collecting, evaluating and/or outputting or providing measured values or measurement results 713, in particular from the sensor apparatus 113, and/or test results and/or other data or values.

The control apparatus 207 preferably comprises an internal clock or time base by means of which the sequence of the test is or can be controlled and/or by means of which test steps that follow temporally one another or that extend over time are controlled or can be controlled by the control apparatus 207.

The control apparatus 207 preferably controls or is designed to control actuators of the analysis device 200 for acting on the cartridge 100 in order to carry out the test.

The actuators are in particular the pump drive 202, the temperature-control apparatuses and/or the valve actuators 205A, B.

The analysis system 1 or analysis device 200 preferably comprises one or more sensors 206A-H.

In particular, fluid sensors 206A are designed or provided to detect liquid fronts and/or flows of fluid in the fluid system 103. Particularly preferably, the fluid sensors 206A are designed to measure or detect, for example optically and/or capacitively, a liquid front and/or the presence, the speed, the mass flow rate/volume flow rate, the temperature and/or another value of a fluid in a channel and/or a cavity, in particular in a respectively assigned sensor portion 116, which is in particular formed by a planar and/or widened channel portion of the fluid system 103.

The fluid sensor 206A preferably measures a fluid entering or leaving the sensor portion 116 and/or a content change or fluid change in the sensor portion 116, and in the process generates a measurement result 706A that corresponds to the fluid entering, the fluid leaving, the content change and/or the fluid change in the sensor portion 116. This measurement result 706A from the fluid sensor 206A can be retrieved by the control apparatus 207 and/or transmitted to the control apparatus 207.

The control apparatus 207 controls or is designed to control the test and/or the actuators, preferably using or taking into account the measurement result 706A from the fluid sensor 206A. In particular, when a content change, an entering fluid, a leaving fluid and/or a fluid change is detected in the sensor portion 116, in particular when a liquid front is detected, the control apparatus 207 influences a program sequence. In this case, for example a check can be carried out or a subsequent step of the test can be controlled, in particular by activating the actuators in a particular and/or differing manner.

Particularly preferably, the sensor portions 116 are each oriented and/or incorporated in the fluid system 103 and/or fluid flows against or through the sensor portions 116 such that, in the operating position of the cartridge 100, fluid flows through the sensor portions 116 in the vertical direction and/or from the bottom to the top, or vice versa, in particular in order to make it possible or easier to accurately detect liquid.

Alternatively or additionally, the analysis device 200 preferably comprises one or more (different, other and/or further) sensors 206B.

Preferably, the other sensor 206B is or comprises a pressure sensor for determining the (relative) air pressure. The other sensor 206B can generate a measurement result 706B, which corresponds in particular to the air pressure. This measurement result 706B can be retrieved by the control apparatus 207 and/or transmitted to the control apparatus 207. The control apparatus 207 controls or is designed to control the test and/or the actuators, preferably using or taking into account the measurement result 706B from the other sensor 206B.

Alternatively or additionally, one or more temperature sensors 206C are provided for detecting the internal temperature and/or the temperature in the interior space 212A of the analysis device 200, in particular the temperature of an atmosphere in the interior space 212A. Alternatively or additionally, one or more temperature sensors 206C are provided for detecting the ambient temperature and/or the temperature of an atmosphere surrounding the analysis device 200 and/or the temperature of one or more of the temperature apparatuses.

The temperature sensor 206C preferably measures a temperature, in particular of the interior space 212A of the analysis device 200, and in the process generates a measurement result 706C that corresponds to the temperature, in particular of the interior space 212A and/or atmosphere of or parts of the interior space 212A. This measurement result 706C from the temperature sensor 206C can be retrieved by the control apparatus 207 and/or transmitted to the control apparatus 207. The control apparatus 207 controls or is designed to control the test and/or the actuators, preferably using or taking into account the measurement result 706C from the temperature sensor 206C.

The analysis device 200 preferably comprises a tilt sensor 206D for detecting the inclination and/or orientation of the analysis device 200 and/or of the cartridge 100. The tilt sensor 206D preferably generates a measurement result 706D that corresponds to the inclination or represents the inclination. In the following, the invention is described in greater detail on the basis of the inclination, and the inclination can also be replaced by the term "measurement result 706D". The tilt sensor 206D is in particular designed and set up to determine the inclination of the analysis device 200 and/or of the cartridge 100 that differs from that in an operating position.

According to one aspect , which can also be implemented independently, the inclination of the analysis device 200 and/or the cartridge 100 is monitored. The inclination is preferably monitored by the analysis device 200, in particular the control apparatus 207, by means of the tilt sensor 206D.

The tilt sensor 206D preferably comprises or is formed by a microelectromechanical system. In particular, this is a spring-mass system, by means of which the excursion of one or more test masses is determined, and the inclination or measurement result 706D is determined on this basis. The tilt sensor 206D can measure the inclination about one or more axes.

The tilt sensor 206D is preferably designed to measure its inclination, and to measure the inclination of the cartridge 100 and/or the analysis device 200 indirectly in that the tilt sensor 206D is coupled to the analysis device 200 and/or the cartridge 100.

As shown in Fig. 1 by way of example, the tilt sensor 206D can be connected or fastened to the housing 212 of the analysis device 200 or to another part of the analysis device 200 in order for it to be possible to measure an inclination of the analysis device 200 and/or the cartridge 100. Alternatively or additionally, the tilt sensor 206D or another tilt sensor is assigned to the cartridge 100, in particular is provided at the cartridge 100, thereon or as a part thereof, in order to measure the inclination of the cartridge 100. The tilt sensor 206D can also be designed to measure both the inclination of the cartridge 100 and the inclination of the analysis device 200.

As already explained at the outset, the term "inclination" is in particular understood to mean a deviation from a vertical direction or from a plane perpendicular to the vertical direction. "Vertical direction" is understood to mean the direction of gravity, as can be determined by a plumb line or plummet, for example.

The analysis device 200 and/or the cartridge 100 preferably has an operating position determined or defined in relation to the vertical direction, the cartridge 100 preferably being oriented at least substantially in the vertical direction by a surface extension thereof or an extension thereof between connections of cavities 108, and/or a base, mid-plane or the like of the analysis device 200 preferably being oriented at least substantially perpendicularly to the vertical direction.

The inclination is thus preferably a deviation in the spatial position or alignment of the cartridge 100 and/or the analysis device 200. Preferably, the cartridge 100 and/or the analysis device 200 is correctly oriented when the main plane of extension of the cartridge 100 extends at least substantially in parallel with the force of gravity and/or vertically in the normal operating position and/or in the analysis device 200, and/or a lower longitudinal side of the cartridge 100 extends perpendicularly to the force of gravity and/or horizontally. However, other concepts are also possible here.

In addition, it is preferable for the cartridge 100 to have a specified or determined position and orientation relative to the analysis device 200 when it is received in the analysis device 200 in order to carry out the test. The inclination of the cartridge 100, which is understood to be a deviation from the operating position, therefore corresponds to the inclination of the analysis device 200, and vice versa. However, other solutions are also possible here. In particular, the cartridge 100 can be orientable with respect to to the inclination within the analysis device 200, in particular such that an inclination is compensated for and/or the cartridge 100 is or can be moved into its operating position.

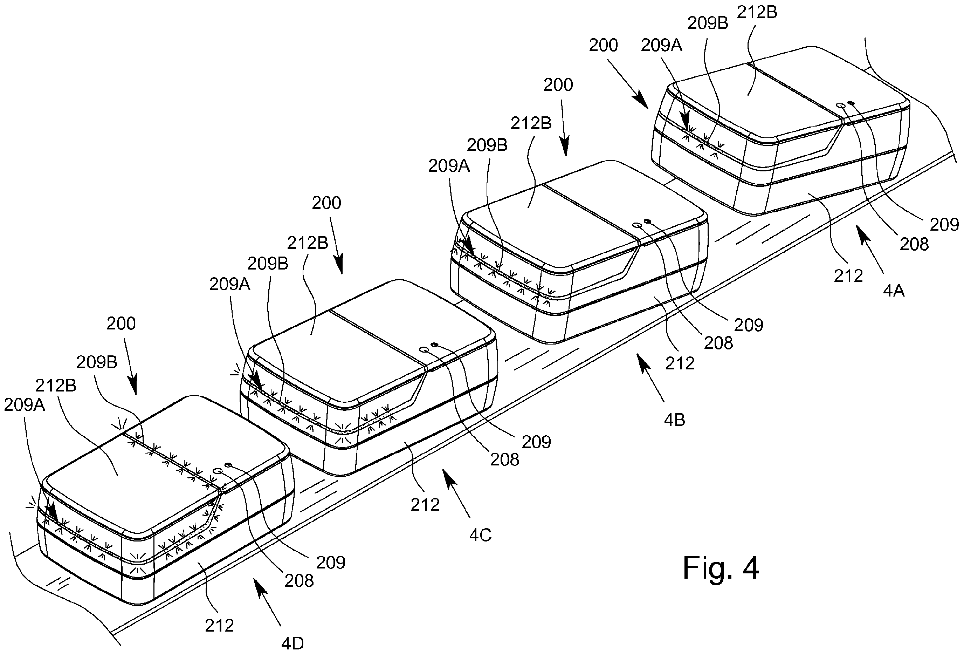

Preferably, the measured inclination or a measurement result 706D corresponding thereto is displayed, in particular by means of the display apparatus 209, as explained in greater detail in the following in conjunction with Fig. 4.

The inclination of the cartridge 100 and/or the analysis device 200 is or can be adapted if necessary by in particular (partially) vertically adjusting a support apparatus 215 of the analysis device 200.

In particular, it is possible for an incorrect inclination or an inclination that negatively affects the test to be measured, and, if necessary, for the orientation of the analysis device 200 to be adapted, in particular by means of a support apparatus 215, preferably such that the cartridge 100 is at least substantially vertically oriented and/or the inclination is compensated for.

Particularly preferably, the inclination is measured, detected and/or output before the test is carried out. This provides for a particularly accurate, rapid and reliable test.

Particularly preferably, if the cartridge 100 and/or the analysis device 200 are oriented incorrectly, in particular so as to be inclined or tilted, a test is blocked or prevented, in particular electronically. Preferably, the test on the sample P is only started or carried out if the cartridge 100 is oriented correctly and/or vertically. This prevents or reduces any potential measurement errors or inaccuracies that result from the cartridge 100 and/or the analysis device 200 being incorrectly oriented.

The term "orientation" is preferably understood to mean the spatial position or alignment, preferably the inclination, of the cartridge 100 and/or the analysis device 200, particularly preferably relative to the force of gravity, in particular in the normal operating position. Preferably, the cartridge 100 and/or the analysis device 200 is correctly oriented when the main plane of extension of the cartridge 100 extends at least substantially in parallel with the force of gravity and/or vertically in the normal operating position and/or in the analysis device, and/or a lower longitudinal side of the cartridge 100 extends perpendicularly to the force of gravity and/or horizontally.

Fig. 4 shows the proposed analysis device 200 in four different inclination positions 4A to 4D, the analysis device 200 being inclined relative to its operating position in the first inclination position 4A and this inclination being reduced in position 4B, being further reduced in position 4C and being reduced in position 4D to such an extent that the analysis device 200 has reached its operating position.

The tilt sensor 206D preferably measures the inclination, and in the process generates a measurement result 706D that corresponds to the inclination of the analysis device 200 and/or of the cartridge 100. This measurement result 706D from the tilt sensor 206D can be retrieved by the control apparatus 207 and/or transmitted to the control apparatus 207. The control apparatus 207 controls or is designed to control the test and/or the actuators, preferably using or taking into account the measurement result 706D from the tilt sensor 206D. In particular, if the inclination is too great, the test is prevented, blocked or interrupted, and/or an error is identified, processed, transmitted and/or signalled. Alternatively or additionally, the test can be controlled taking into account the inclination, and potential effects or deviations brought about by the inclination can be compensated for.

The test is preferably blocked from starting depending on the inclination or can be blocked from starting depending on the inclination, in particular if the inclination reaches or exceeds a start threshold value 526.

The start threshold value 526 is preferably less than 40°, in particular less than 35° and/or preferably more than 20°, in particular more than 25°, particularly preferably between 28° and 32°, more particularly preferably at least essentially 30°.

Preferably, the test is only started if the inclination of the analysis device 200 and/or cartridge 100 with respect to the operating position is below 40°, in particular below 35°, particularly preferably below 30°.

Alternatively or additionally, the test can be interrupted depending on the inclination, preferably if the inclination reaches or exceeds an interruption threshold value 527.

The interruption threshold value 527 preferably differs from the start threshold value 526. Here, the interruption threshold value 527 can correspond to a greater inclination than the start threshold value 526. It is thus preferable for the start of the test to be blocked if the inclinations are smaller, but once the test is underway, it is only interrupted if the inclinations are greater.

The test is preferably interrupted when the inclination of the analysis device 200 and/or cartridge 100 is above 45° or 60° and/or the interruption threshold value 527 is preferably more than 45° or 60°, and/or less than 90° or 75°.

An error can be detected, signalled and/or stored depending on the inclination, in particular when a threshold value 525 is exceed. Said threshold value 525 can for example be the start threshold value 526, the interruption threshold value 527 or another threshold value 525 between the start threshold value 526 and the interruption threshold value 527.

Using the tilt sensor 206D, the current or present inclination of the analysis device 200 or the cartridge 100 relative to the operating position and/or an inclination provided in an operating position is preferably determined as the inclination of the analysis device 200 or the cartridge 100. In particular, the measurement result 706D that corresponds to the inclination and is used for or as the inclination is measured.

It is clear that the result of the comparison of an inclination in the operating position with a current inclination matters, but the comparison itself can be carried out in different ways and in different reference systems.

The inclination, i.e. in particular the measurement result 706D, is preferably compared with a threshold value 525, in particular with the interruption threshold value 527 and/or the start threshold value 526. The test and/or the display apparatus 209 can be controlled using the result of the comparison. Alternatively or additionally, other events can also be triggered on the basis of the result. The inclination, the measurement result 706D and/or the result of the comparison can be transmitted to the operating instrument 400. The operating instrument 400 can carry out the comparison, display and/or signal the inclination, or cause other events to take place.

The threshold value 525, the start threshold value 526 and/or the interruption threshold value 527 can be determined, set or changed, preferably depending on the cartridge 100 using which the test is intended to be carried out, and/or on the cartridge identifier 100C of the cartridge 100, and/or on control information 510 for carrying out the test using the cartridge 100.

The threshold value 525, the start threshold value 526 and/or the interruption threshold value 527 can be individual to the respective tests, cartridges 100, batches of cartridges 100 and/or kinds of cartridges 100, and may be stored, retrievable and/or retrieved.

Using the same or different cartridges 100, the analysis device 200 preferably supports different tests, one of the tests being selected and the start threshold value 526 and/or the interruption threshold value 527 being determined or being able to be determined depending on the selected test.

The support apparatus 215 is preferably designed to support the analysis device 200 or a housing 212 of the analysis device 200 on a surface underneath. The support apparatus 215 preferably comprises one or more support elements 215A, in particular feet or the like.

One or more of the support elements 215A may be adjustable, in particular height-adjustable, in order to change or compensate for an inclination of the analysis device 200 and/or the cartridge 100. In particular, the support elements 215A are height-adjustable feet or the like.

In one aspect, the support apparatus 215, in particular the support elements 215A, can be controlled and/or driven. In particular, the support elements 215A are automatically drivable support elements 215A, in particular support elements 215A that are height-adjustable and/or are movable relative to the housing 212. In particular, the support elements 215A can be extended out of the housing 212 and/or can be retracted into the housing 212, such that the inclination of the analysis device 200 can be changed thereby.

Preferably, one or more support elements 215A can be controlled depending on the inclination of the analysis device 200 and/or the cartridge 100. In particular, the control apparatus 207 is designed to receive measurement results 706D from the tilt sensor 206D and to control or regulate one or more automatic support elements 215 thereby, such that the inclination is reduced and/or in order to incline the analysis device 200 and/or the cartridge 100 towards its operating position and/or bring said analysis device 200 and/or cartridge 100 into the operating position. Alternatively or additionally, the analysis device 200 can be designed to signal an inclination and/or to signal an inclination of the analysis device 200 towards and/or into the operating position, in particular by optically and/or acoustically signalling or outputting signals. Said signals are preferably output using an output apparatus or display apparatus 209 of the analysis device 200 and/or an output apparatus 410 of the operating instrument 400.

The analysis device 200 may comprise an acceleration sensor 206E. The acceleration sensor 206E is preferably designed to determine an acceleration of the analysis device 200, in particular an acceleration in the vertical and/or horizontal direction with respect to the operating position.

The acceleration sensor 206E preferably measures the acceleration, and in the process generates a measurement result 706E that corresponds to the acceleration of the analysis device 200 and/or of the cartridge 100. This measurement result 706E from the acceleration sensor 206E can be retrieved by the control apparatus 207 and/or transmitted to the control apparatus 207. The control apparatus 207 controls or is designed to control the test and/or the actuators, preferably using or taking into account the measurement result 706E from the acceleration sensor 206E. In particular, if the acceleration is too great, the test is prevented, blocked or interrupted, and/or an error is identified, processed, transmitted and/or signalled.

The analysis device 200 may comprise a humidity sensor 206F for determining the (relative) atmospheric humidity and/or the dew point of the atmosphere inside or in the interior space 212A and/or outside the analysis device 200.

The humidity sensor 206F preferably measures the (relative) atmospheric humidity and/or the dew point, and in the process generates a measurement result 706F that corresponds to the (relative) atmospheric humidity and/or the dew point of the atmosphere in the analysis device 200 and/or the surroundings of the analysis device 200. This measurement result 706F from the humidity sensor 206F can be retrieved by the control apparatus 207 and/or transmitted to the control apparatus 207. The control apparatus 207 controls or is designed to control the test and/or the actuators, preferably using or taking into account the measurement result 706F from the humidity sensor 206F. In particular, if the (relative) atmospheric humidity is too high and/or if the dew point is approached or reached, the test is prevented, blocked and/or interrupted, or an error is identified, processed, transmitted and/or signalled.

The analysis device 200 may comprise a position sensor 206G for determining the position or location, for example by means of a GPS sensor. The position sensor 206G is preferably designed to determine the location of the analysis device 200 in space, in particular on the Earth's surface, and/or to output the geographical position, the location and/or the coordinates of the analysis device 200.

The position sensor 206G preferably measures the position, in particular the geographical position, of the analysis device 200, and in the process generates a measurement result 706G that corresponds to the position or geographical position. This measurement result 706G from the position sensor 206G can be retrieved by the control apparatus 207 and/or transmitted to the control apparatus 207. The control apparatus 207 controls or is designed to control the test and/or the actuators, preferably using or taking into account the measurement result 706G from the position sensor 206G.

In particular by means of the position sensor 206G and/or the other sensor 206B, in particular the pressure sensor, the geographical elevation or a measured value 706B, 706G corresponding thereto can be determined. The analysis device 200 can be controlled on the basis of the measured value 706B, 706G, preferably as described in conjunction with the inclination, in particular by correspondingly comparing threshold values, blocking or enabling the test, and the like.

The analysis device 200 may comprise a cartridge sensor 206H for determining or checking the position or alignment of the cartridge 100 in or with respect to the analysis device 200. In particular, the cartridge sensor 206H is designed to detect an incorrect position of the cartridge 100 in the analysis device 200. Alternatively or additionally, the cartridge sensor 206H is designed to detect and/or verify the correct and/or operating position of the cartridge 100 in the analysis device 200.

The cartridge sensor 206H preferably measures the position of the cartridge 100 in the analysis device 200, and in the process generates a measurement result 706H that corresponds to the position or alignment of the cartridge 100 in the analysis device 200. This measurement result 706H from the cartridge sensor 206H can be retrieved by the control apparatus 207 and/or transmitted to the control apparatus 207. The control apparatus 207 controls or is designed to control the test and/or the actuators, preferably using or taking into account the measurement result 706H from the cartridge sensor 206H. In particular, if the cartridge 100 is incorrectly positioned in the analysis device 200, the test is prevented or blocked and/or the cartridge 100 is automatically ejected from the analysis device 200 or the like. Alternatively or additionally, the test is enabled if it is detected that the cartridge 100 is in the correct operating position in the analysis device 200.

The control apparatus 207 preferably controls or regulates the pump drive 202, the temperature-control apparatuses 204A-C and/or the valve actuators 205A, B, in particular taking into account or depending on the desired test and/or measured values or measurement results 713 from the sensor arrangement or sensor apparatus 113 and/or sensors 206A-H.

The control apparatus 207 controls or is designed to control the test and/or the actuators, preferably using or taking into account the measurement result 706A from the fluid sensor 206A. In particular, when a content change, an entering fluid, a leaving fluid or a fluid change is identified in the sensor portion 116, in particular when a liquid front is detected, the control apparatus 207 influences a program sequence. In this case, for example a check can be carried out or a subsequent step of the test can be controlled, in particular by activating the actuators in a particular and/or differing manner.

The flows of fluid are controlled in particular by accordingly activating the pump or pump apparatus 112 and actuating the valves 115A, 115B.

Particularly preferably, the pump drive 202 comprises a stepper motor, or a drive calibrated in another way, such that desired metering can be achieved, at least in principle, by means of appropriate activation.

Additionally or alternatively, the fluid sensors 206A are used to detect liquid fronts or flows of fluid, in particular in cooperation with the assigned sensor portions 116, in order to achieve the desired fluidic sequence and the desired metering by accordingly controlling the pump or pump apparatus 112 and accordingly activating the valves 115A, 115B.

Optionally, the analysis system 1 or analysis device 200 comprises an input apparatus 208, such as a keyboard, a touch screen or the like, and/or a display apparatus 209, such as a screen.

The analysis system 1 or analysis device 200 preferably comprises at least one interface 210, for example for controlling, for communicating and/or for outputting measured data or test results and/or for linking to other devices, such as a printer, an external power supply or the like. This may in particular be a wired or wireless interface 210.

The analysis system 1 or analysis device 200 preferably comprises a power supply 211, preferably a battery or an accumulator, which is in particular integrated and/or externally connected or connectable.

Preferably, an integrated accumulator is provided as a power supply 211 and can be (re)charged by an external charging device (not shown) via a connection 211A and/or is interchangeable.

The analysis system 1 or analysis device 200 preferably comprises a housing 212, all the components and/or some or all of the apparatuses preferably being integrated in the housing 212. Particularly preferably, the cartridge 100 can be inserted or slid into the housing 212, and/or can be received by the analysis device 200, through an opening 213 which can in particular be closed, such as a slot or the like.

The analysis system 1 or analysis device 200 is preferably portable or mobile. Particularly preferably, the analysis device 200 weighs less than 25 kg or 20 kg, particularly preferably less than 15 kg or 10 kg, in particular less than 9 kg or 6 kg.

The fluidic, in particular pneumatic, coupling between the cartridge 100 and the analysis device 200 will be explained in greater detail in the following, it being possible for the following aspects to be implemented independently from the preceding aspects.

As already explained, the analysis device 200 can preferably be pneumatically linked to the cartridge 100, in particular to the sensor arrangement or sensor apparatus 113 and/or to the pump apparatus 112.

Particularly preferably, the analysis device 200 is designed to supply the cartridge 100, in particular the sensor arrangement or sensor apparatus 113 and/or the pump apparatus 112, with a working medium, in particular gas or air.

Preferably, the working medium can be compressed and/or pressurised in the analysis device 200 or by means of the analysis device 200.

Preferably, the analysis device 200 comprises a pressurised gas supply 214 for this purpose, in particular a pressure generator or compressor, preferably in order to compress and/or pressurise the working medium.

The pressurised gas supply 214 is preferably integrated in the analysis device 200 or the housing 212 and/or can be controlled or feedback controlled by means of the control apparatus 207. The pressurised gas supply 214 can also, at least in part, be formed on or by the cartridge 100.

Preferably, the pressurised gas supply 214 is electrically operated or can be operated by electrical power. In particular, the pressurised gas supply 214 can be supplied with electrical power by means of the power supply 211.

The analysis device 200 or pressurised gas supply 214 is preferably designed to compress the working medium to a pressure of more than 100 kPa, particularly preferably more than 150 kPa or 250 kPa, in particular more than 300 kPa or 350 kPa, and/or of less than 1 MPa, particularly preferably less than 900 kPa or 800 kPa, in particular less than 700 kPa and/or to feed said medium to the cartridge 100 at said pressure.

Preferably, air can be drawn in, in particular from the surroundings, as the working medium by means of the analysis device 200 or pressurised gas supply 214. In particular, the analysis device 200 or pressurised gas supply 214 is designed to use the surroundings as a reservoir for the working medium or the air. However, other solutions are also possible here, in particular those in which the analysis device 200 or pressurised gas supply 214 comprises a preferably closed or delimited reservoir, such as a tank or container, comprising the working medium, and/or is connected or connectable thereto.

Preferably, the analysis device 200 or pressurised gas supply 214 comprises an inlet, the working medium in particular being able to be drawn in and/or conducted in the pressurised gas supply 214 via the inlet.

Preferably, the analysis device 200 or pressurised gas supply 214 comprises a filter, the filter preferably being integrated in the inlet and/or it preferably being possible for the working medium to be filtered by means of the filter and/or it preferably being possible for particles to be separated from the working medium by means of the filter.

The filter is preferably designed as a micro filter or as a fine particulate air filter. Preferably, particles having a particle diameter of more than 10 µm, particularly preferably more than 8 µm or 9 µm, in particular more than 6 µm or 7 µm, more particularly preferably more than 4 µm or 5 µm, can be separated by means of the filter, the particle diameter preferably being the maximum or average diameter of the respective particles. This ensures that the channels or lines in the cartridge that convey the working medium do not become contaminated or clogged and/or that no undesired pressure loss occurs.

The analysis device 200 or pressurised gas supply 214 preferably comprises a connection element 214A, in particular in order to pneumatically connect the analysis device 200 and/or pressurised gas supply 214 to the cartridge 100.

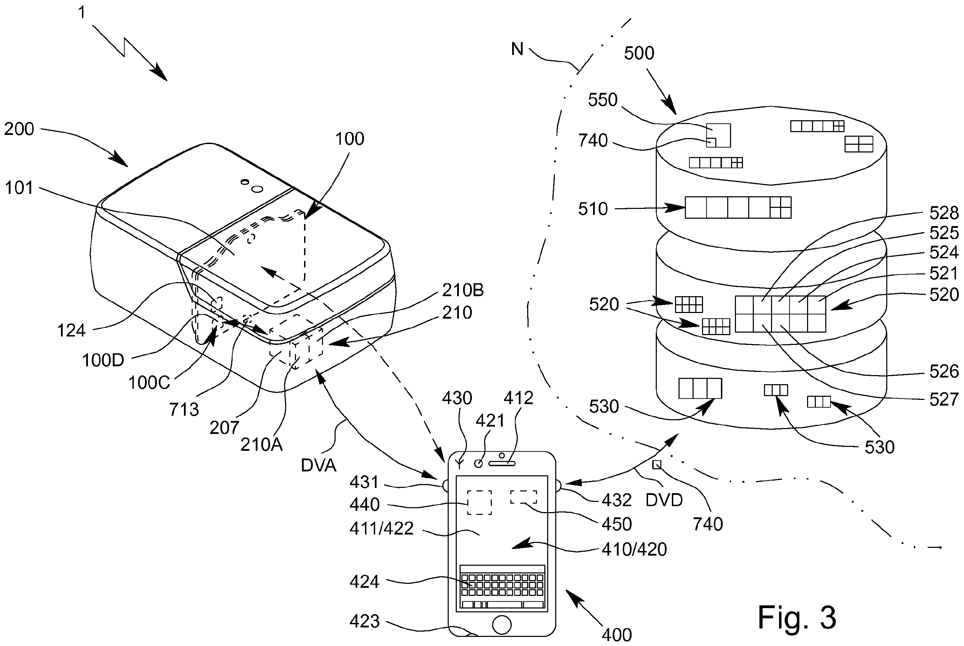

Fig. 3 is a schematic view of the proposed analysis system 1 for testing an in particular biological sample P, comprising the analysis device 200 for receiving the cartridge 100 and subsequently carrying out the test using the received cartridge 100, and an operating instrument 400 for the analysis device 200.

The operating instrument 400 is preferably designed to control the analysis device 200. Alternatively or additionally, the operating instrument 400 can receive or retrieve information, in particular (measurement) results such as measured values, from the analysis device 200. In particular, the operating instrument 400 is a mobile terminal device such as a smartphone, a tablet or the like.

The operating instrument 400 is preferably implemented or provided so as to be physically separated from the analysis device 200. The operating instrument 400 can preferably be separated and/or disconnected from the analysis device 200 physically and/or with respect to a data connection.

The operating instrument 400 can preferably be wirelessly connected to the analysis device 200. A data connection DVA can thus be established between the analysis device 200 and the operating instrument 400. However, the data connection DVA can in principle also be established in another manner, for example wired.

It is preferable for the operating instrument 400 to also be operational when separated or disconnected from the analysis device 200, in particular for carrying out evaluations or for other purposes. Alternatively or additionally, the analysis device 200 is also operational when separated or disconnected from the operating instrument 400, in particular for continuing a test.

Particularly preferably, the operating instrument 400 comprises an interface 430 for establishing data connections DVA, DVD. The interface 430 and/or the operating instrument 400 in particular comprises what is referred to as an analysis device interface 431 that is designed to establish the preferably wireless data connection DVA to the analysis device 200. This can, for example, be a radio interface, WPAN interface, Bluetooth interface and/or a Bluetooth module or the like.

The interface 210 of the analysis device 200 preferably corresponds to the interface 430 and/or the analysis device interface 431 of the operating instrument 400, in particular such that the data connection DVA between the operating instrument 400 and the analysis device 200 can be established. The interface 210 of the analysis device 200 and the analysis device interface 431 preferably support the same data transmission method and/or radio transmission method or radio standard, in particular WLAN or WPAN methods such as Bluetooth, NFC, Zigbee or the like.

Particularly preferably, the interface 210 of the analysis device 200 and the analysis device interface 431 make possible or facilitate what is known as an ad-hoc connection. In this case, the data connection DVA is established preferably automatically when the devices, i.e. the operating instrument 400 and the analysis device 200, are within range of one another. In other words, the operating instrument 400 and the analysis device 200 each comprise a wireless data interface 430, 210, respectively, which are designed to jointly establish an ad-hoc data connection between the operating instrument 400 and the analysis device 200, preferably such that, when the operating instrument 400 and the analysis device 200 approach one another in space, the data connection DVA therebetween is automatically established and is preferably displayed by means of the operating instrument 400.

The data connection DVA is preferably a point-to-point connection. The data connection DVA connects the analysis device 200 to the operating instrument 400 preferably directly and/or without any interposed networks. It is possible for the operating instrument 400 to establish data connections DVA to different analysis devices 200 simultaneously or in succession. Alternatively or additionally, it is possible for one analysis device 200 to establish data connections DVA to a plurality of operating instruments 400 simultaneously or in succession.

In order to control the test, it is preferable for precisely one data connection DVA to be provided between the analysis device 200 to be controlled and the operating instrument 400 controlling the analysis device 200, and/or for control information 510 to be received and/or accepted or to be acceptable and/or receivable and/or for measurement results 713 to be transmitted or to be transmittable only via precisely one data connection DVA between the analysis device 200 to be controlled and the operating instrument 400 controlling the analysis device 200.

The analysis device 200 preferably comprises a receiver 210A for, preferably wirelessly, receiving the control information 510 from the operating instrument 400. Preferably, the interface 210 comprises the receiver 210A, via which signals, in particular control information 510, are or can be received from the operating instrument 400.

Alternatively or additionally, the analysis device 200 and/or the interface 210 comprises a transmitter 210B, via which data, in particular results such as measurement results 713 from the sensor apparatus 113, are or can be sent, particularly preferably to the operating instrument 400.

The interfaces 210, 431 preferably correspond to one another such that they support the same data transmission standard and/or radio standard, in particular Bluetooth, WLAN or the like. These interfaces are particularly preferably interfaces 210, 431 which make possible what is known as an ad-hoc connection, the data connection DVA preferably being established automatically when the devices, i.e. the operating instrument 400 and the analysis device 200, are within range of one another.

The analysis system 1 preferably further comprises a database 500 or the database 500 is assigned to the analysis system 1. The database 500 is preferably an external database 500 that is implemented or provided so as to be physically separated from the operating instrument 400 and/or from the analysis device 200. In principle, however, it is not impossible for the database 500 to be provided or implemented such that it can be directly linked, in particular to the operating instrument 400, or to be provided or implemented by the operating instrument 400.

The operating instrument 400 can access the database 500 via a data connection DVD. For this purpose, the operating instrument 400 and/or the interface 430 can comprise a database interface 432 by means of which the database 500 can be accessed, in particular via a network N. The network N may be the Internet or another data network. It is also preferable for the operating instrument 400 to be able to establish the data connection DVD to the database 500 via a wireless interface, in particular WLAN, WPAN, mobile communications or the like. However, in principle, other solutions are also possible here.

Particularly preferably, the operating instrument 400 comprises different interfaces 430 that are independent of one another for establishing data connections DVA, DVD to the analysis device 200 and to the database 500, the analysis device 200 (as a peripheral device of the operating instrument 400) being designed to communicate exclusively with or via the operating instrument 400.

The analysis system 1, in particular the database 500, preferably comprises control information 510 by means of which the analysis device 200 can be controlled in order to carry out a test.

The control information 510 preferably defines the actuation of the actuators of the analysis device 200 in a particular manner, such that the sample P is tested in the cartridge 100. In particular, actuators for carrying out the test can be or are controlled using the control information 510 such that said actuators act on the cartridge 100 and/or the sample P. These actuators are in particular the pump drive 202 and/or one or more temperature-control apparatuses 204A-C and/or one or more valve actuators 205A, B. The control information 510 preferably comprises parameters and/or instructions for carrying out one or more steps of the method for testing the sample P explained above.

Preferably, the analysis system 1 comprises calibration information 520 that can be stored in the database 500 and/or can be retrieved from the database 500. The calibration information 520 is preferably capable of influencing the test of the sample P, in particular depending on the specific cartridge 100, on a cartridge batch of the specific cartridge 100 and/or on the specific test.

The calibration information 520 is in particular default or basic settings, parameters and/or threshold values for sensors such as the sensor apparatus 113 of the cartridge 100, for one or more of the sensor(s) 206A-H of the analysis device 200 and/or for one or more of the actuators.

Calibration information 520 can be used in addition to control information 510 for carrying out the test, the calibration information 520 preferably influencing or specifying the control information 510. The calibration information 520 can be or can form the control information 510 or a part of the control information 510, even if this is not explicitly mentioned in the following.

The analysis device 200 can be calibrated and/or configured by calibration information 520 that can form part of the control information 510 or can be provided separately. For this purpose, the calibration information 520 can be determined, retrieved and/or transmitted to the analysis device 200 by means of the operating instrument 400.

In one example, fluid sensor calibration information 521 is provided which influences setting and/or evaluation of the fluid sensor 206A. The fluid sensor calibration information 521 is preferably dependent on the test to be carried out, the phase of the test and/or expected effects of a content change in a sensor portion 116 during the test sequence, and/or contains various specifications which are dependent thereon.

Alternatively or additionally, tilt sensor calibration information 524 can be provided, preferably one or more threshold values 525, in particular a start threshold value 526 for blocking the start of a test if said threshold value is exceeded, and/or an interruption threshold value 527 for interrupting the test and/or for processing errors if said threshold is exceeded.

Alternatively or additionally, sensor arrangement calibration information 528 can be provided, by means of which properties of the sensor arrangement 113 or sensor apparatus 113 are or can be set. In particular, it is provided that the sensor arrangement calibration information 528 is transmitted or can be transmitted to the sensor arrangement 113 or sensor apparatus 113 by the analysis device 200, and that the sensor arrangement 113 or sensor apparatus 113 carries out or is designed to carry out a measurement taking into account the sensor arrangement calibration information 528.

The proposed analysis system 1 preferably comprises evaluation information 530 which is stored in the database 500 and/or is retrievable or can be retrieved from the database 500. The evaluation information 530 is preferably designed to be able to interpret measurement results 713 that originate from the cartridge 100, in particular from the sensor apparatus 113.

The control information 510 and/or the evaluation information 530 particularly preferably comprises instructions, preferably in the form of an algorithm and/or for controlling a process on or using a processor or controller. The instructions preferably form a module that can be or is implemented by the analysis device 200 and/or the operating instrument 400, as a result of which the behaviour of the analysis device 200 and/or the operating instrument 400 can be or is changed.

The instructions are in particular commands, machine code, pre-compiled source code or source code. The instructions preferably form a module-like software component, in particular a plugin. The instructions can be designed to form and/or to replace a module of the operating instrument 400 and/or of the analysis device 200. For this purpose, the control information 510 and/or the evaluation information 530 can comprise a (software) interface for coupling or implementation by the control apparatus 207 and/or an evaluation module 440 of the operating instrument 400.

The control information 510 particularly preferably comprises or forms a module of the control apparatus 207 that can be exchanged, preferably in terms of software. This module preferably contains instructions such as logic commands, loops and the like for controlling the test, in particular in the form of a computer program or computer program product to be executed by the analysis device 200 and/or the control apparatus 207. The control information 510 can be or form, in particular as a plugin, an exchangeable part of the control apparatus 207.

An evaluation module 440 is preferably formed by the operating instrument 400 or the operating instrument 400 comprises the evaluation module 440. By means of the evaluation module 440, measurement results 713 read out from the sensor apparatus 113 are evaluated preferably using the evaluation information 530 retrieved from the database 500 and/or the evaluation module 440 is designed for this purpose.

The evaluation information 530 particularly preferably comprises or forms a module of the evaluation apparatus 440 that can be exchanged, preferably in terms of software. This module preferably contains instructions such as logic commands, loops and the like for controlling the evaluation of measurement results 713, in particular in the form of a computer program or computer program product to be executed by the operating instrument 400 and/or the evaluation module 440. The evaluation information 530 can be or form, in particular as a plugin, an exchangeable part of the evaluation module 440. The computer program product preferably is a non-transitory computer-readable media.

Alternatively or additionally, the instructions can comprise parameters for configuring the control apparatus 207 and/or the evaluation module 440. These parameters are preferably provided in addition to the instructions, for example for the analysis device 200 in the form of or comprising the calibration information 520. Alternatively, the control information 510 and/or evaluation information 530 can however also merely comprise parameters and/or other information for the control and/or evaluation.

The database 500 preferably comprises a results memory 550 in which results can be stored and/or saved.

Within the meaning of the present invention, the term "database" should preferably be understood in a broad sense and also incorporates multi-part databases in particular. Therefore, in principle, the database 500 can be provided in different physical units or at different locations and/or can be composed of a plurality of subdata-bases.

The operating instrument 400 can preferably be separated and/or disconnected from the analysis device 200 with respect to a data connection and/or physically. For this purpose, the analysis device 200 can initially be connected to the operating instrument 400 by the data connection DVA being established.

In order to control the test and/or the analysis device 200, the operating instrument 400 can retrieve control information 510 from the database 500 and transmit said information to the analysis device 200 in unaltered or altered form.

The operating instrument 400 is preferably designed to evaluate measurement results 713 which can preferably be generated by the sensor apparatus 113 of the cartridge 100 while the sample P is being tested. For this purpose, it is provided that measurement results 713, which can originate from a sensor apparatus 113 of the cartridge 100 and/or which can be transmitted from the analysis device 200 to the operating instrument 400, are or can be evaluated in the operating instrument 400. For this purpose, the operating instrument 400 can retrieve or receive the evaluation information 530 from the database 500 and, using this evaluation information 530, evaluate the measurement results 713, in particular in the evaluation module 440 of the operating instrument 400.

The operating instrument 400 preferably comprises a memory 450. The memory 450 can be used to store, at least temporarily, control information 510, calibration information 520 and/or evaluation information 530, or the operating instrument 400 and the memory 450 can be designed for this purpose. Alternatively or additionally, evaluation results 740, that have been or can be generated from the measurement results 713 by means of the operating instrument 400, can be stored in the memory 450.

In one example, the operating instrument 400 comprises an output apparatus 410, preferably an in particular touch-sensitive screen or display 411 and/or a speaker 412. Alternatively or additionally, the operating instrument 400 comprises an input apparatus 420, in particular a camera 421, a touchpad 422, a microphone 423 and/or a keyboard 424.

The operating instrument 400 is preferably designed to display an operating interface or a user interface via the output apparatus 410, in particular the screen or display 411, or to provide in another way operating elements for controlling the test and/or the analysis device 200, and/or to output a status or other information relating to the test. Alternatively or additionally, commands can be received via the input apparatus 420, by means of which the operating instrument 400 starts, configures and/or controls the test of the sample P in a manner corresponding to the commands.

Preferably, the transmission of commands and/or information to the analysis device 200 is triggered via the input apparatus 420 or can be triggered by the input apparatus 420.

In particular, transmission of the control information 510 from the operating instrument 400 to the analysis device 200 can be initiated or controlled via the input apparatus 420. Alternatively or additionally, the analysis device 200 can be controlled in order to receive the cartridge 100 and/or to start the test, preferably using the control information 510 and/or a command received via the input apparatus 420. The operating instrument 400 is therefore preferably designed to transmit to the analysis device 200 control information 510 for receiving or ejecting the cartridge 100. In this case, a cartridge 100 can in particular be inserted only when the operating instrument 400 is connected to the analysis device 200, whereupon the operating instrument 400 can verify the cartridge 100 and can eject said cartridge or block a test if an error, such as incompatibility, is detected.

Alternatively or additionally, the operating instrument 400 is designed to transmit control information 510 for starting the test to the analysis device 200. The test is thus preferably started only by a command originating from the operating instrument 400. The analysis device 200 itself preferably does not comprise a user interface for generating a start command or for causing the test to start. This task is preferably reserved for the operating instrument 400.

The cartridge 100 preferably comprises at least one cartridge identifier 100C which corresponds to the cartridge 100 and/or to a batch with which the cartridge 100 is associated.

The cartridge identifier 100C is in particular a piece of information that is specific to the relevant cartridge 100, is in particular unique and/or is designed to uniquely identify the cartridge 100, such as an identification code which is assigned to the relevant cartridge 100 and makes it possible for said cartridge to be identified in a preferably unique manner.

Alternatively or additionally, the cartridge identifier 100C makes it possible to assign the cartridge 100 to a production cycle and/or to a batch of particular cartridges 100. A batch is preferably characterised in that cartridges 100 are produced in the same continuous production cycle and/or are produced having the same components, in particular having the same sensor apparatuses 113 and/or the same reagents and the like. There is preferably a plurality of batches which can differ from one another with regard to production periods, batches of starting materials used and the like, for example.

The cartridge identifier 100C can be stored and/or saved in a memory means 100D of the cartridge 100. The memory means 100D can be a barcode 124, an NFC tag and/or a memory which is provided in the sensor apparatus 113, is connected to the sensor apparatus 113 or is assigned to the sensor apparatus 113, or another apparatus for storing code or the like.

The cartridge identifiers 100C are preferably assigned to the respective cartridges 100. In particular, the cartridge identifier 100C is formed by the cartridge 100, connected thereto and/or arranged thereon.

The analysis system 1 can comprise one or a plurality of cartridges 100 which can each preferably be distinguished from one another by means of at least one cartridge identifier 100C and/or which are assigned to a batch.

Fig. 4 shows the proposed analysis device 200 at different degrees of inclination when tilted or inclined relative to a surface underneath or to a horizontal reference surface, i.e. the reference surface oriented perpendicularly to the vertical direction.

In one aspect of the present invention, which can also be implemented independently, the analysis device 200 comprises a light strip 209A as a display apparatus 209.

The analysis device 200 is preferably designed to signal an operating status and/or a requirement of the analysis device 200 by means of the display apparatus 209, in particular by means of the light strip 209A. As an alternative or in addition to the light strip 209A, another display apparatus 209 may also be provided, for example a status light, a screen or the like.

The light strip 209A may comprise a plurality of, preferably more than five or more than ten, light sources 209B, which are preferably arranged along the light strip 209A. The light sources 209B are preferably light-emitting diodes. However, other solutions are also possible here.

It is preferable for the light strip 209A to comprise light sources 209B that are at least substantially evenly spaced, the spacing preferably being less than 10 cm, preferably less than 5 cm. In principle, however, the light strip 209A may also have varying spacing, for example if due to the housing 212 of the analysis device 200 it is required to bridge edges or other structures.

The light strip 209A can be activated and/or deactivated, preferably in portions, by the plurality of light sources 209B or in another manner such that it only emits light in regions.

However, other solutions are also possible here, for example those in which the light strip 209A is or can be activated and deactivated only in its entirety, it being possible for different signals to be output by flashing, colour selection and/or colour sequences. For example, an analysis device 200 can signal a non-ready-to-use state by means of a red colour, or the like.

Preferably, the light strip 209A comprises light sources 209B and apparatuses such as optical waveguides and/or diffusers to allow for an at least substantially continuously illuminated light strip 209A. The light strip 209A is however preferably also designed to illuminate only in part or in portions and/or simultaneously in different colours. This makes it possible, in a particularly effective manner, to signal different states on the basis of colours or partial operation or illumination.

The light strip 209A may be more than 10 cm long, preferably more than 20 or 30 cm long. Preferably, the light strip 209A has a length that exceeds its width by a factor of more than 10, preferably a factor of more than 20 or 30. It is preferable here for the light strip 209A to be designed to be narrow, for example to be less than 10 mm wide, preferably less than 5 mm wide and/or less than a tenth or a twentieth of the height, width and/or length of the analysis device 200.

The light strip 209A is preferably provided on or so as to be recessed into the housing 212 of the analysis device 200. The light strip 209A preferably faces the outside, emits light to the outside and/or is placed onto or inserted into the housing 212 such that the light strip 209A is visible from the outside.

The light strip 209A preferably forms a self-contained or closed structure. A self-contained or closed form of the light strip 209A is preferably already provided when the light strip 209A does not have an end, for example in that an end and a start of the light strip 209A directly adjoin one another and/or when the light strip 209A forms a loop, meaning that a continuous structure is formed. However, other solutions are also possible here.

Particularly preferably, the light strip 209A is arranged peripherally. It is not entirely necessary for said light strip 209A to be arranged peripherally on a side wall of the housing 212 of the analysis device 200 so as to be symmetrical to a central axis of the analysis device 200, although this is possible in principle and is preferable.

Furthermore, it is not impossible for the light strip 209A to have breaks, for example if the light strip 209A is formed by a plurality of light sources 209B that form the light strip 209A by being arranged so as to be aligned with one another at least in portions. It is possible here for the light sources 209B to be spaced apart from one another, and in this case reference is preferably also made to a light strip 209A.

In the example shown in Fig. 4, the light strip 209A is provided around a preferably displaceable cover or displaceable housing part 212B and/or is provided along the opening 213 in the analysis device 200, or is arranged, preferably peripherally, on said opening 213. In particular, the cover or displaceable housing part 212B is a drawer-like cover that is at least substantially horizontally displaceable in the operating position. By displacing the cover or housing part 212B, the opening 213 in the analysis device 200 can be released, opened or closed in order for the cartridge 100 to be inserted into or received in the analysis device 200, in particular to protect against the ingress of dirt or other foreign matter or impurities.

In particular, the light strip 209A is arranged along an edge of the opening 213 or of the movable housing part 212B of the housing 212. In this case, the light strip 209A is provided on the movable housing part 212B of the housing 212, on the fixed part of the housing 212, or on both the movable part and the fixed part of the housing 212.

Particularly preferably, the housing 212 comprises a drawer-like mechanism, by means of which the opening 213 can be closed and/or opened. The drawer-like mechanism may be formed by or may comprise the movable part 212B of the housing 212. The movable part 212B of the housing 212 and/or of the drawer-like mechanism can preferably be moved so as to be closed or open in order to close or open the opening 213. The light strip 209A may be provided along the drawer-like mechanism, in particular along a (displaceable) edge or a (displaceable) end of the housing part 212B of the housing 212 that is displaceable or shiftable or can be moved in another manner.

By means of the display apparatus 209, in particular the light strip 209A, the analysis device 200 can be designed to output or signal an operating state such as alignment, an error, a wireless connection status and/or a test process and/or a requirement, such as the need to be cleaned or the need for maintenance.

In particular, a status of the wireless data connection DVA to an operating instrument 400, a status of the power supply 211 and/or anenergy storage means 211B of the analysis device 200 and/or a status relating to the test on the sample P and/or a need for action relating to boundary conditions of the analysis device, such as alignment, position, inclination, movement and/or temperature, are signalled.

For this purpose, measurement results 706 from one or more of the sensors 206AH can be used in particular by the control apparatus 207 to control the display apparatus 209, in particular the light strip 209A, or the analysis device 200 and/or the control apparatus 207 is designed for this purpose.

In particular by calculation, threshold-value comparison, assignment, in particular by means of an assignment table or assignment function, or the like, the control apparatus 207 can calculate or determine in some other way in which manner, in particular in which regions, portions, with which colours, brightnesses, intensities or the like, the display apparatus 209, in particular the light strip 209A, is to be operated. Subsequently, the control apparatus 207 can control the display apparatus 209, in particular the light strip 209A, in the determined manner in order to signal the inclination, the orientation of the inclination, the degree of inclination, whether the operating position has been reached, or other operating states and/or requirements by means of the display apparatus 209, in particular the light strip 209A.

Signalling or output is carried out by the output apparatus 209, in particular by means of the light strip 209A, preferably using colours and/or by flashing and/or by the light strip 209A only being activated in part or by the emission of light coded in another manner.

For example, the light strip 209A can generate a chase (i.e. the light sources 209B light up in sequence) when the test is being carried out or can be controlled accordingly. Alternatively or additionally, in the event of a warning or of an error being detected, the light strip 209A can be controlled such that it flashes by alternately being completely illuminated and completely switched off, i.e. not illuminated.

In a preferred embodiment, the light strip 209A is controlled by means of the inclination. In particular, the tilt sensor 206D or a measurement result 706D originating therefrom is evaluated by the control apparatus 207 and the light strip 209A is controlled depending on the measurement result 706D. In this case, the light strip 209A can be used to form an electronic spirit level, i.e. to signal an inclination, in particular in comparison with a target value and/or the operating position, and/or to guide a user how to reduce the inclination and/or how to orient the analysis device 200 and/or the cartridge 100 into the opening position. Here, the signalling can preferably allow conclusions to be drawn on the degree and/or direction of the inclination, meaning that it is possible to orient the analysis device 200 without additional tools in a simple and interactive manner.

For this purpose, the light strip 209A is controlled, preferably by the control apparatus 207, such that the light strip 209A is actuated unevenly or non-uniformly depending on the inclination, in particular if the inclination exceeds a threshold value.

Particularly preferably, the light strip 209A is operated unevenly or non-uniformly or asymmetrically such that an illuminated portion of the light strip 209A is assigned to a region or portion of the analysis device 200 that needs to be raised relative to other portions of the analysis device 200 in order to reach the operating position, while a non-illuminated portion of the light strip 209A is assigned to a region of the analysis device 200 that needs to be lowered relative to other portions of the analysis device 200.

In principle, the signalling can also be reversed, such that a region or portion of the analysis device 200 that needs to be lowered relative to other portions in order to reach the operating position is signalled by means of the illuminated light strip 209A, while another portion of the analysis device 200 that needs to be raised relative to the other portion or other portions of the analysis device 200 is signalled by means of the non-illuminated light strip 209A.

In a development of this aspect, it is preferable for the light strip 209A to display the extent or degree of inclination of the analysis device 200 and/or the cartridge 100 relative to the operating position. Here, it may be provided that, in a correct orientation in which the analysis device 200 and/or the cartridge 100 is arranged in the operating position, the light strip 209A is completely illuminated or is not illuminated, is lit up or goes out.

When inclined relative to the operating position, the light strip 209A is preferably deactivated in part depending on the inclination direction, such that the light strip 209A preferably displays the inclination direction. Alternatively or additionally, the light strip 209A is activated or deactivated in larger or smaller regions or portions depending on the extent or degree of inclination, so that this represents the extent or degree of inclination. The extent or degree of inclination may alternatively or additionally be signalled by specific light intensities and/or colours.

The display by the light strip 209A thus changes depending on the extent or degree of inclination and/or on the inclination direction in order to signal the extent and the inclination direction.

As an alternative or in addition to the display explained above, by activating and deactivating the light strip 209A in portions, it is also possible to signal the inclination, the inclination direction and/or the extent of inclination using colour coding, in particular regions of the display apparatus 209, in particular the light strip 209A, illuminated in different colours.

In the example shown, in Fig. 4 in position 4A, the analysis device 200 is inclined relative to the indicated reference surface, which is oriented at least substantially horizontally and/or perpendicularly to the vertical direction. In position 4A, this is signalled by the light strip 209A only being activated in portions, at the highest point. However, the signalling may, alternatively or additionally, also be coloured or may be carried out by actuating the light strip 209A in reverse, i.e. by activating the light strip 209A in part or in portions at the lowest point or the like.

In Fig. 4 in position 4B, the analysis device 200 is inclined to a lesser extent than in position 4A. In the preferred embodiment, this is also signalled by the light strip 209A, in particular by the portion of the light strip 209A that is activated or deactivated or colour-coded or signalling the inclination in another manner being enlarged or reduced in size. This behaviour can be continued as the inclination is reduced further, as shown in position 4C, until the analysis device 200 has reached the operating position, as shown in position 4D by way of example.

In the operating position, the display apparatus 209, in particular the light strip 209A, can signal the correct orientation and/or correct inclination of the analysis device 200 in particular by means of a completely activated or deactivated or completely and/or evenly actuated light strip 209A.

In a particularly preferred embodiment, the more selectively and/or unevenly and/or non-uniformly and/or asymmetrically the light strip 209A is actuated, the greater the inclination. It is preferable here for the light strip 209A to be actuated non-uniformly or unevenly such that it is actuated symmetrically relative to the inclination direction. Alternatively or additionally, the unevenness or non-uniformity or asymmetry of the actuation or operation of the light strip 209A is preferably most pronounced transversely to an inclination direction, i.e. the differences from the opposing sides of a central axis of the light strip 209A extending transversely to the inclination direction are the greatest.

For example, the light strip 209A is activated at the highest point and is deactivated at the lowest point, or vice versa. This means that the light strip 209A is activated unevenly or non-uniformly and is at least substantially symmetrical relative to the line connecting the highest point to the lowest point. In this way, the display apparatus 209, in particular the light strip 209A, can signal in which manner, in which direction and the extent to which the inclination needs to be changed in order to move the analysis device 200 and/or the cartridge 100 into the operating position.

In general, the analysis device 200, the cartridge 100 or in particular the sensor apparatus 113 may measure, detect or identify the one or more analytes A by means of specific bonding, in particular by means of capture molecules and/or of means of electrochemical detection such as redox cycling, or the like, preferably performed on the cartridge 100 and/or in the sensor apparatus 113. Preferably, the capture molecules are arranged or immobilized on a sensor array or on sensor fields or electrodes of the sensor apparatus 113. In particular, an immuno-assay or a protein assay for detecting or identifying a protein and/or a nucleic-assay for detecting or identifying a nucleic-acid sequence can be or is realized.

Alternatively or additionally, measurements without specific bonding and/or without electrochemical detection can be used or performed, preferably in or by the analysis device 200 and/or cartridge 100. Such measurements can include an optical measurement, impedance measurement, capacitance measurement, spectrometric measurement, mass spectrometric measurement, or the like. For this purpose, the analysis device 200 or cartridge 100 may comprise an optical spectrometer and/or allow optical measurements of the treated or untreated sample P. Thus, it is possible to measure, detect or identify other or further analytes A, compounds, material characteristics, or the like of the sample P, e.g. within the cartridge 100 or any other sample carrier. These alternative or additional measurements can be used or processed and/or evaluated in a similar manner as described or differently.

**List of reference signs:**

| | | | |
|---|---|---|---|
| 1 | analysis system | 203A | contact element |
| | | 204A | reaction temperature-control apparatus |
| 100 | cartridge | | |
| 100C | cartridge identifier | 204B | intermediate temperature-control apparatus |
| 100D | memory means | | |
| 101 | main body | 204C | sensor temperature-control apparatus |
| 102 | cover | | |
| 103 | fluid system | 205A | (valve) actuator for 115A |
| 104 | receiving cavity | 205B | (valve) actuator for 115B |
| 104A | connection | 206A | fluid sensor |
| 104B | inlet | 206B | other sensor |
| 104C | outlet | 206C | temperature sensor |
| 104D | intermediate connection | 206D | tilt sensor |
| 105 | metering cavity | 206E | acceleration sensor |
| 106A-G | intermediate cavity | 206F | humidity sensor |
| 107 | mixing cavity | 206G | position sensor |
| 108A-E | storage cavity | 206H | cartridge sensor |
| 109A | first reaction cavity | 207 | control apparatus |
| 109B | second reaction cavity | 208 | input apparatus |
| 109C | third reaction cavity | 209 | display apparatus |
| 110 | intermediate temperature-control cavity | 209A | light strip |
| | | 209B | light source |
| 111 | collection cavity | 210 | interface |
| 112 | pump apparatus | 210A | receiver |
| 113 | sensor apparatus | 210B | transmitter |
| 114 | channel | 211 | power supply |
| 114A | bypass | 211A | connection |
| 115A | initially closed valve | 211 B | energy storage means |
| 115B | initially open valve | 212 | housing |
| 116 | sensor portion | 212A | interior space |
| 124 | barcode | 212B | housing part |
| | | 213 | opening |
| 200 | analysis device | 214 | pressurised gas supply |
| 201 | receptacle | 214A | connection element |
| 202 | pump drive | 215 | support apparatus |
| 203 | connection apparatus | 215A | support element |
| 400 | operating instrument | 706C | measurement result from the temperature sensor |
| 410 | output apparatus | 706D | measurement result from the tilt sensor |
| 411 | display | | |
| 412 | speaker | 706E | measurement result from the acceleration sensor |
| 420 | input apparatus | | |
| 421 | camera | 706F | measurement result from the humidity sensor |
| 422 | touchpad | | |
| 423 | microphone | 706G | measurement result from the position sensor |
| 424 | keyboard | | |
| 430 | interface | 706H | measurement result from the cartridge sensor |
| 431 | analysis device interface | | |
| 432 | database interface | 713 | measurement result from the sensor apparatus |
| 440 | evaluation module | | |
| 450 | memory | | |
| | | 740 | evaluation result |
| 500 | database | | |
| 510 | control information | A | analyte |
| 520 | calibration information | DVA | data connection analysis device - operating instrument |
| 521 | fluid sensor calibration information | | |
| | | DVD | data connection database - operating instrument |
| 524 | calibration information (tilt | | |
| | sensor) | F(1-5) | liquid reagent(s) |
| 525 | threshold value (tilt sensor) | N | (data) network |
| 526 | start threshold value (tilt sensor) | P | sample |
| | | S(1-10) | dry reagent(s) |
| 527 | interruption threshold value (tilt sensor) | | |
| 528 | calibration information (sensor arrangement) | | |
| 530 | evaluation information | | |
| 550 | results memory | | |
| 706A | measurement result from the fluid sensor | | |
| 706B | measurement result from the other sensor | | |

## Claims

1. Method for controlling an analysis device (200), the analysis device (200) being designed for testing an in particular biological sample (P), it being possible for the sample (P) to be received in a cartridge (100), and the analysis device (200) being designed for receiving the cartridge (100) and subsequently carrying out the test using the received cartridge (100),
**characterised**
**in that** the analysis device (200) comprises a light strip (209A) as a display apparatus (209), by means of which the analysis device (200) signals an operating status and/or a requirement, wherein the light strip (209A) is provided at least substantially peripherally and/or all round on the analysis device (200).

2. Method according to claim 1, **characterised in that** the need to be cleaned and/or the need for maintenance and/or an error and/or a wireless connection status and/or a test process, is/are output.

3. Method according to claim 1 or 2, **characterised in that** operating states or requirements of the analysis device (200) are signalled by light being emitted that is colour-coded and/or coded by flashing.

4. Method according to any one of the preceding claims, **characterised in that** a status of a wireless data connection (DVA) to an operating instrument (400), and/or a status of a power supply (211) and/or an energy storage means (211 B), and/or a status relating to the test is/are signalled.

5. Method according to any one of the preceding claims, **characterised in that** the inclination of the analysis device (200) and/or the cartridge (100) is monitored and the inclination of the analysis device (200) is signalled.

6. Method according to any one the preceding claims, **characterised in that** the inclination of the analysis device (200) and/or the cartridge (100) is monitored and how to incline the analysis device (200) towards or into an operating position is signalled.

7. Method according to claim 6, **characterised in that** the light strip (209A) functions as an electronic spirit level, in particular wherein an inclination above a threshold value (525) is represented by an asymmetrical or non-uniform or uneven display on the light strip (209A), and/or an inclination below a threshold value (525) is represented by a symmetrical or uniform or even display on the light strip (209A).

8. Analysis system (1) for testing an in particular biological sample (P),
the analysis system (1) comprising a cartridge (100) for receiving the sample (P), the cartridge (100) comprising a fluid system for conveying the sample (P),
the analysis system (1) comprising an analysis device (200) for receiving the cartridge (100) and subsequently carrying out the test using the received cartridge (100), preferably wherein the analysis system (1) is configured to carry out the method according to any one of the preceeding claims,
**characterised**
**in that** the analysis device (200) comprises a light strip (209A) as a display apparatus (209) and is configured to signal an operating status and/or a requirement by means of the light strip (209A), wherein the light strip (209A) is provided at least substantially peripherally and/or all round on the analysis device (200).

9. Analysis system according to claim 8, **characterised in that** the light strip (209A) is arranged on a surface of the analysis device (200) that is visible from the outside, and/or **in that** the light strip (209A) is arranged on a side wall of the analysis device (200).

10. Analysis system according to claim 8 or 9, **characterised in that** the light strip (209A) has a strip-like, elongate, and/or self-contained shape.

11. Analysis system according to any one of claims 8 to 10, **characterised in that** the analysis device (200) is configured to output by means of the light strip (209A) a need to be cleaned and/or a need for maintenance and/or an error and/or a wireless connection status and/or a test process.

12. Analysis system according to any one of claims 8 to 11, **characterised in that** the analysis device (200) is configured to signal operating states or requirements of the analysis device (200) by light being emitted by the light strip (209A) that is colour-coded and/or coded by flashing.

13. Analysis system according to any one of claims 8 to 12, **characterised in that** the analysis device (200) is configured to signal a status of a wireless data connection (DVA) to an operating instrument (400), and/or a status of a power supply (211) and/or an energy storage means (211B), and/or a status relating to the test.

14. Analysis system according to any one of claims 8 to 13, **characterised in that** the analysis system (1) is configured to monitor the inclination of the analysis device (200) and/or the cartridge (100) and to signal at least one of the inclination of the analysis device (200) and how to incline the analysis device (200) towards or into an operating position.

15. Computer program product comprising program code means which, when executed, cause the following steps to be implemented:
controlling an analysis device (200), the analysis device (200) being designed for testing an in particular biological sample (P), it being possible for the sample (P) to be received in a cartridge (100), and the analysis device (200) being designed for receiving the cartridge (100) and subsequently carrying out the test using the received cartridge (100), and
signaling an operating status and/or a requirement by means of a light strip (209A) as a display apparatus (209) of the analysis device (200), the light strip (209A) being provided at least substantially preferably and/or all round on the analysis device (200).

## Patentansprüche

1. Verfahren zur Steuerung eines Analysegeräts (200), wobei das Analysegerät (200) zur Untersuchung einer insbesondere biologischen Probe (P) ausgebildet ist, wobei die Probe (P) in eine Cartridge (100) aufnehmbar ist und wobei das Analysegerät (200) zur Aufnahme der Cartridge (100) und anschließenden Durchführung der Untersuchung mit der aufgenommenen Cartridge (100) ausgebildet ist,
**dadurch gekennzeichnet,**
**dass** das Analysegerät (200) als Anzeigeeinrichtung (209) ein Lichtband (209A) aufweist, mit dem das Analysegerät (200) einen Betriebsstatus und/oder ein Erfordernis signalisiert, wobei das Lichtband (209A) an dem Analysegerät (200) zumindest im Wesentlichen umfangsseitig und/oder rundum vorgesehen ist.

2. Verfahren nach Anspruch 1, **dadurch gekennzeichnet, dass** ein Reinigungsbedarf und/oder ein Wartungsbedarf und/oder ein Fehler und/oder ein Funkverbindungsstatus und/oder ein Untersuchungsvorgang, ausgegeben wird/werden.

3. Verfahren nach Anspruch 1 oder 2, **dadurch gekennzeichnet, dass** Betriebszustände oder Erfordernisse des Analysegeräts (200) durch farblich und/oder durch Blinken codierte Emission von Licht signalisiert werden.

4. Verfahren nach einem der voranstehenden Ansprüche, **dadurch gekennzeichnet, dass** ein Status einer drahtlosen Datenverbindung (DVA) mit einer Bedienvorrichtung (400), und/oder ein Status einer Energieversorgung (211) und/oder eines Energiespeichers (211B), und/oder ein Status betreffend die Untersuchung, signalisiert wird/werden.

5. Verfahren nach einem der voranstehenden Ansprüche, **dadurch gekennzeichnet, dass** die Neigung des Analysegeräts (200) und/oder der Cartridge (100) überwacht und die Neigung des Analysegeräts (200) signalisiert wird.

6. Verfahren nach einem der voranstehenden Ansprüche, **dadurch gekennzeichnet, dass** die Neigung des Analysegeräts (200) und/oder der Cartridge (100) überwacht wird und signalisiert wird, wie das Analysegerät (200) in Richtung oder in eine Betriebsposition zu neigen ist.

7. Verfahren nach einem der voranstehenden Ansprüche, **dadurch gekennzeichnet, dass** das Lichtband (209A) als elektronische Wasserwaage fungiert, insbesondere wobei eine Neigung oberhalb eines Schwellwerts (525) durch eine asymmetrische oder uneinheitliche oder ungleichmäßige Anzeige auf dem Lichtband (209A) repräsentiert wird, und/oder eine Neigung unterhalb eines Schwellwerts (525) durch eine symmetrische oder einheitliche oder gleichmäßige Anzeige auf dem Lichtband (209A) repräsentiert wird.

8. Analysesystem (1) zur Untersuchung einer insbesondere biologischen Probe (P),
wobei das Analysesystem (1) eine Cartridge (100) zur Aufnahme der Probe (P) aufweist,
wobei die Cartridge (100) ein Fluidsystem zur Förderung der Probe (P) aufweist,
wobei das Analysesystem (1) ein Analysegerät (200) zur Aufnahme der Cartridge (100) und anschließender Durchführung der Untersuchung mit der aufgenommenen Cartridge (100) aufweist, vorzugsweise wobei das Analysesystem (1) zur Durchführung des Verfahrens nach einem der voranstehenden Ansprüche ausgebildet ist,
**dadurch gekennzeichnet,**
**dass** das Analysegerät (200) als Anzeigeeinrichtung (209) ein Lichtband (209A) aufweist und dazu ausgebildet ist, mit dem Lichtband (209A) einen Betriebsstatus und/oder ein Erfordernis zu signalisieren, wobei das Lichtband (209A) an dem Analysegerät (200) zumindest im Wesentlichen umfangsseitig und/oder rundum vorgesehen ist.

9. Analysesystem nach Anspruch 8, **dadurch gekennzeichnet, dass** das Lichtband (209A) an einer von außen sichtbaren Oberfläche des Analysegeräts (200) angeordnet ist, und/oder dass das Lichtband (209A) an einer Seitenwand des Analysegeräts (200) angeordnet ist.

10. Analysesystem nach Anspruch 8 oder 9, **dadurch gekennzeichnet, dass** das Lichtband eine bandartige, längliche, und/oder in sich geschlossene Form aufweist.

11. Analysesystem nach einem der Ansprüche 8 bis 10, **dadurch gekennzeichnet, dass** das Analysegerät (200) dazu ausgebildet ist, mit dem Lichtband (209A) einen Reinigungsbedarf und/oder einen Wartungsbedarf und/oder einen Fehler und/oder einen Funkverbindungsstatus und/oder einen Untersuchungsvorgang auszugeben.

12. Analysesystem nach einem der Ansprüche 8 bis 11, **dadurch gekennzeichnet, dass** das Analysegerät (200) dazu ausgebildet ist, Betriebszustände oder Erfordernisse des Analysegeräts (200) durch vom Lichtband (209A) emittiertes Licht zu signalisieren, das farblich codiert und/oder durch Blinken codiert ist.

13. Analysesystem nach einem der Ansprüche 8 bis 12, **dadurch gekennzeichnet, dass** das Analysegerät (200) dazu ausgebildet ist, einen Status einer drahtlosen Datenverbindung (DVA) mit einer Bedienvorrichtung (400), und/oder einen Status einer Energieversorgung (211) und/oder eines Energiespeichers (211B), und/oder einen Status betreffend die Untersuchung zu signalisieren.

14. Analysesystem nach einem der Ansprüche 8 bis 13, **dadurch gekennzeichnet, dass** das Analysesystem (1) dazu ausgebildet ist, die Neigung des Analysegeräts (200) und/oder der Cartridge (100) zu überwachen und mindestens eines von der Neigung des Analysegeräts (200) und wie das Analysegerät (200) in Richtung oder in eine Betriebsposition zu neigen ist zu signalisieren.

15. Computerprogrammprodukt aufweisend Programmcodemittel, die, wenn sie ausgeführt werden, die Ausführung der folgenden Schritte bewirken:
Steuern eines Analysegeräts (200), wobei das Analysegerät (200) zur Untersuchung einer insbesondere biologischen Probe (P) ausgebildet ist, wobei die Probe (P) in eine Cartridge (100) aufnehmbar ist und wobei das Analysegerät (200) zur Aufnahme der Cartridge (100) und anschließenden Durchführung der Untersuchung mit der aufgenommenen Cartridge (100) ausgebildet ist, und
Signalisieren eines Betriebsstatus und/oder ein Erfordernisses mittels eines Lichtband (209A) als Anzeigeeinrichtung (209) des Analysegeräts (200), wobei das Lichtband (209A) an dem Analysegerät (200) zumindest im Wesentlichen umfangsseitig und/oder rundum vorgesehen ist.

## Revendications

1. Procédé de commande d'un dispositif d'analyse (200), le dispositif d'analyse (200) étant conçu pour tester un échantillon (P), en particulier biologique, l'échantil-Ion (P) pouvant être reçu dans une cartouche (100), et le dispositif d'analyse (200) étant conçu pour recevoir la cartouche (100) et effectuer ensuite le test en utilisant la cartouche (100) reçue,
**caractérisé**
**en ce que** le dispositif d'analyse (200) comprend une bande lumineuse (209A) en tant qu'appareil d'affichage (209), au moyen duquel le dispositif d'analyse (200) signale un état de fonctionnement et/ou une exigence, la bande lumineuse (209A) étant prévue au moins essentiellement à la périphérie et/ou tout autour du dispositif d'analyse (200).

2. Procédé selon la revendication 1, **caractérisé en ce que** le besoin de nettoyage et/ou le besoin de maintenance et/ou une erreur et/ou un état de connexion sans fil et/ou un processus de test, est/sont émis.

3. Procédé selon la revendication 1 ou 2, **caractérisé en ce que** les états de fonctionnement ou les exigences du dispositif d'analyse (200) sont signalés par l'émission d'une lumière codée par couleur et/ou codée par clignotement.

4. Procédé selon l'une des revendications précédentes, **caractérisé en ce qu'**un état d'une connexion de données sans fil (DVA) à un instrument de commande (400), et/ou un état d'une alimentation électrique (211) et/ou d'un moyen de stockage d'énergie (211B), et/ou un état relatif au test est/sont signalé(s).

5. Procédé selon l'une des revendications précédentes, **caractérisé en ce que** l'inclinaison du dispositif d'analyse (200) et/ou de la cartouche (100) est surveillée et l'inclinaison du dispositif d'analyse (200) est signalée.

6. Procédé selon l'une des revendications précédentes, **caractérisé en ce que** l'inclinaison du dispositif d'analyse (200) et/ou de la cartouche (100) est surveillée et la manière d'incliner le dispositif d'analyse (200) vers ou dans une position de fonctionnement est signalée.

7. Procédé selon la revendication 6, **caractérisé en ce que** la bande lumineuse (209A) fonctionne comme un niveau à bulle électronique, en particulier une inclinaison supérieure à une valeur seuil (525) étant représentée par un affichage asymétrique ou non uniforme ou inégal sur la bande lumineuse (209A), et/ou une inclinaison inférieure à une valeur seuil (525) étant représentée par un affichage symétrique ou uniforme ou égal sur la bande lumineuse (209A).

8. Système d'analyse (1) pour tester un échantillon (P), en particulier biologique, le système d'analyse (1) comprenant une cartouche (100) pour recevoir l'échantillon (P), la cartouche (100) comprenant un système de fluide pour transporter l'échantillon (P),
le système d'analyse (1) comprenant un dispositif d'analyse (200) pour recevoir la cartouche (100) et effectuer ensuite le test en utilisant la cartouche (100) reçue, de préférence le système d'analyse (1) étant configuré pour effectuer le procédé selon l'une des revendications précédentes,
**caractérisé**
**en ce que** le dispositif d'analyse (200) comprend une bande lumineuse (209A) en tant qu'appareil d'affichage (209) et est configuré pour signaler un état de fonctionnement et/ou une exigence au moyen de la bande lumineuse (209A), la bande lumineuse (209A) étant prévue au moins essentiellement à la périphérie et/ou tout autour du dispositif d'analyse (200).

9. Système d'analyse selon la revendication 8, **caractérisé en ce que** la bande lumineuse (209A) est disposée sur une surface du dispositif d'analyse (200) qui est visible de l'extérieur, et/ou **en ce que** la bande lumineuse (209A) est disposée sur une paroi latérale du dispositif d'analyse (200).

10. Système d'analyse selon la revendication 8 ou 9, **caractérisé en ce que** la bande lumineuse (209A) a une forme de bande, allongée et/ou fermée.

11. Système d'analyse selon l'une des revendications 8 à 10, **caractérisé en ce que** le dispositif d'analyse (200) est configuré pour émettre au moyen de la bande lumineuse (209A) un besoin de nettoyage et/ou un besoin de maintenance et/ou une erreur et/ou un état de connexion sans fil et/ou un processus de test.

12. Système d'analyse selon l'une des revendications 8 à 11, **caractérisé en ce que** le dispositif d'analyse (200) est configuré pour signaler des états de fonctionnement ou des exigences du dispositif d'analyse (200) par la lumière émise par la bande lumineuse (209A) qui est codée par couleur et/ou codée par clignotement.

13. Système d'analyse selon l'une des revendications 8 à 12, **caractérisé en ce que** le dispositif d'analyse (200) est configuré pour signaler un état d'une connexion de données sans fil (DVA) à un instrument de commande (400), et/ou un état d'une alimentation électrique (211) et/ou d'un moyen de stockage d'énergie (211B), et/ou un état relatif au test.

14. Système d'analyse selon l'une des revendications 8 à 13, **caractérisé en ce que** le système d'analyse (1) est configuré pour surveiller l'inclinaison du dispositif d'analyse (200) et/ou de la cartouche (100) et pour signaler au moins l'une de l'inclinaison du dispositif d'analyse (200) et la manière d'incliner le dispositif d'analyse (200) vers ou dans une position de fonctionnement.

15. Produit programme d'ordinateur comprenant des moyens de code de programme qui, lorsqu'ils sont exécutés, conduisent à mettre en oeuvre des étapes suivantes :
commander un dispositif d'analyse (200), le dispositif d'analyse (200) étant conçu pour tester un échantillon (P), en particulier biologique, l'échantillon (P) pouvant être reçu dans une cartouche (100), et le dispositif d'analyse (200) étant conçu pour recevoir la cartouche (100) et effectuer ensuite le test en utilisant la cartouche (100) reçue, et
signaler un état de fonctionnement et/ou une exigence au moyen d'une bande lumineuse (209A) en tant qu'appareil d'affichage (209) du dispositif d'analyse (200), la bande lumineuse (209A) étant prévue au moins essentiellement à la périphérie et/ou tout autour du dispositif d'analyse (200).
